Europäisches Patentamt

European Patent Office

Office européen des brevets

(19)

(11) **EP 1 477 179 A1**

(12) **EUROPEAN PATENT APPLICATION**
published in accordance with Art. 158(3) EPC

(43) Date of publication:
**17.11.2004 Bulletin 2004/47**

(51) Int Cl.⁷: **A61K 35/84**, A61P 35/00,
A61P 35/04, A61P 37/04,
A61P 43/00, A23L 1/30

(21) Application number: **03707010.9**

(22) Date of filing: **24.02.2003**

(86) International application number:
**PCT/JP2003/001979**

(87) International publication number:
**WO 2003/070264 (28.08.2003 Gazette 2003/35)**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **22.02.2002 JP 2002047021
09.04.2002 JP 2002106632**

(71) Applicant: **Kureha Chemical Industry Co., Ltd.
Tokyo 103-8552 (JP)**

(72) Inventors:
• **EBINA, Takusaburo
  Sendai-shi, Miyagi 980-0873 (JP)**
• **MATSUNAGA, Kenichi
  Tokorozawa-shi, Saitama 359-1142 (JP)**

(74) Representative:
**Minderop, Ralph H., Dr. rer. nat. et al
COHAUSZ & FLORACK
Patent- und Rechtsanwälte
Bleichstrasse 14
40211 Düsseldorf (DE)**

(54) **ANION EXCHANGE RESIN ADSORBED FRACTION, IMMUNOPOTENTIATOR AND PROMOTER FOR RECOVERY FROM LOADED STRESS ORIGINATING IN MATSUTAKE MUSHROOM**

(57) An adsorption fraction of a liquid mixture by an anion exchange resin, wherein the liquid mixture is obtainable by mixing a hot water extract of a mycelium of *Tricholoma matsutake* FERM BP-7304 with an alkaline solution extract of a mycelial residue obtained when preparing the hot water extract, is disclosed. Further, an immune enhancing agent and an agent for promoting a recovery from stress comprising the above-described novel adsorption fraction as an active ingredient, are disclosed.

EP 1 477 179 A1

SEQUENCE LISTING

<110> Kureha Chemical Industry Co., Ltd.

<120> Fraction adsorbed by an anion exchange resin from Tricholoma matsutake, immune enhancing agent, and agent for promoting recovery from stress

<130> KRH-679

<150> JP 2002-047021
<151> 2002-02-22

<150> JP 2002-106632
<151> 2002-04-09

<160> 4

<170> PatentIn version 3.1

<210> 1
<211> 22
<212> DNA
<213> Mus sp.

<400> 1
cgtgctcatg gctggtgcaa ag                                                    22

<210> 2
<211> 18
<212> DNA
<213> Mus sp.

<400> 2
ttatctgcgt tcttgggc                                                         18

<210> 3
<211> 20
<212> DNA
<213> Mus sp.

<400> 3

```
tggaatccag tggcatgaaa                                    20


<210>  4
<211>  25
<212>  DNA
<213>  Mus sp.


<400>  4
taaaacgcag ctcagtaaca gtccg                              25
```

**Description**

TECHNICAL FIELD

The present invention relates to a novel fraction adsorbed by an anion exchange resin from extracts of *Tricholoma matsutake*, an immune enhancing agent and an agent for promoting a recovery from stress containing the same. The immune enhancing agent or agent for promoting a recovery from stress according to the present invention may be administered as a medicament or in various forms, for example, eatable or drinkable products such as health foods (preferably functional foods) or feeds. The term "foods" as used herein include drinks. Further, the agent of the present invention may be administered in the form of an oral hygienic composition which is temporarily kept in the mouth but then spat out, retaining almost none of the components, for example, a dentifrice, a mouthwash agent, a chewing gum, or a collutorium, or in the form of an inhalation drawn in through the nose.

BACKGROUND ART

The present inventors demonstrated that an administration of biological response modifiers (BRM) into a primary tumor alleviated not only the primary tumor but also a metastatic tumor, by a "double grafted tumor system" in mice developed by part of the present inventors and collaborators (Ebina, Biotherapy, 14, 379-394, 2000; Ebina et al., Gan to Kagaku-ryouhou (Jpn J. Cancer & Chemotherapy), 18, 1812-1816, 1991; and Ebina et al., Biotherapy, 12, 495-500, 1998). A mere primary tumor can be extirpated by an operation. However, in cases of metastatic foci (particularly small metastatic foci not detectable with the examination), if a primary tumor is extirpated, the metastatic foci begins to proliferate, and thus the treatment cannot cure cancer. In other words, an important problem in cancer treatment is the treatment of metastatic foci.

Hitherto, it was believed that a local administration of an agent to cancer was not preferred because of the spread of tumor cells. However, the present inventors found that not only a primary focus but also metastatic foci was regressed by local administration of a BRM to cancer (Ebina et al., Jpn.J.Cancer Res., 77, 1034-1042, 1986). In the study, a protein-bound polysaccharide PSK, extracted from mycelia of *Coriolus versicolor* (FR.) Quél., was able to cure not only a primary tumor but also metastatic foci, whereas lentinan, β-glucan purified from fruit bodies of *Lentinus edodes*, did not exhibit an antitumor activity. Further, cepharanthin, extracted from Stephania cepharantha Hayata, exhibited an antitumor activity to right and left tumors [Ebina et al., Gan to Kagaku-ryouhou (Jpn J. Cancer & Chemotherapy), 28, 211-215, 2001], but the purified alkaloid exhibited a weak antitumor activity.

It was found from the above results that, among BRMs, purified substances obtained by Western medicine exhibited a weak or little antitumor activity, whereas extracts containing an active ingredient were superior thereto [Ebina et al., Gan to Kagaku-ryouhou (Jpn J. Cancer & Chemotherapy), 28, 1515-1518, 2001]. Further, a new concept of a third medicine or integrated medicine, which was different from a mixture of several kinds of extracts, such as Chinese drugs in Chinese medicine, was formulated [Ebina et al., Igaku no Ayumi (Journal of Clinical and Experimental Medicine), 194, 690-692, 2000].

Further, it is known that *Tricholoma matsutake* (S.Ito & Imai) Sing. contains many physiologically active substances. For example, Japanese Examined Patent Publication (Kokoku) No. 57-1230 and Japanese Patent No. 2767521 disclose various antitumor substances contained in *Tricholoma matsutake*. The above Japanese Examined Patent Publication (Kokoku) No. 57-1230 discloses that emitanine-5-A, emitanine-5-B, emitanine-5-C, and emitanine-5-D, which are separated and purified from a liquid extract obtained by extracting a broth of *Tricholoma matsutake* mycelia with hot water or a diluted alkaline solution, exhibits an activity of inhibiting a proliferation of sarcoma 180 cells. The above Japanese Patent No. 2767521 discloses that a protein with a molecular weight of 0.2 to 0.21 million (a molecular weight of a subunit = 0.1 to 0.11 million) which is separated and purified from an extract of *Tricholoma matsutake* fruit bodies with water exhibits an antitumor activity.

Further, the present inventors found that a hot water extract of *Tricholoma matsutake* or an alkaline solution extract of *Tricholoma matsutake*, or an adsorption fraction of a hot water extract of *Tricholoma matsutake* or an alkaline solution extract of *Tricholoma matsutake* by an anion exchange resin exhibits an immuno-enhancing activity (WO01/49308).

The present inventors made an intensive search for another substance having an immuno-enhancing activity, and as a result, newly found that a partially purified fraction derived from mycelia obtained by culturing the specific *Tricholoma matsutake* strain by the specific culturing method exhibited such an immuno-enhancing activity. Further, the present inventors found that the fraction was different from known fractions, such as the adsorption fraction by an anion exchange disclosed in WO01/49308, with respect to physicochemical properties, and that the fraction exhibited an activity of promoting a recovery from stress. This present invention is based on the above findings.

DISCLOSURE OF THE INVENTION

Therefore, the object of the present invention is to provide a novel fraction adsorbed (hereinafter referred to as adsorption fraction) by an anion exchange resin from *Tricholoma matsutake*, a novel immune enhancing agent, and a novel agent for promoting a recovery from stress.

The above object can be solved by the present invention, i.e., an adsorption fraction of a liquid mixture by an anion exchange resin, wherein the liquid mixture is obtainable by mixing a hot water extract of a mycelium of *Tricholoma matsutake* FERM BP-7304 with an alkaline solution extract of a mycelial residue obtained when preparing the hot water extract, and

> (a) the content of carbohydrates in the adsorption fraction is 60 to 72% as a glucose equivalent determined by a phenol-sulfuric acid method, and
> (b) the content of proteins in the adsorption fraction is 28 to 40% as an albumin equivalent determined by a copper-Folin method.

Further, the present invention relates to an immune enhancing agent comprising the above-mentioned adsorption fraction as an active ingredient.

The present invention relates to an immune enhancing composition comprising the adsorption fraction and a pharmaceutically acceptable carrier.

The present invention relates to an immune enhancing health food comprising the adsorption fraction alone or, optionally, with one or more food components.

According to a preferred embodiment of the immune enhancing health food of the present invention, the health food is a functional food.

The present invention relates to a method for an immune enhancement, comprising administering to a subject in need thereof the adsorption fraction in an amount effective therefor.

The present invention relates to use of the adsorption fraction in the manufacture of an immune enhancing composition or health food.

Further, the present invention relates to an agent for treating or preventing metastatic foci, comprising the adsorption fraction as an active ingredient.

The present invention relates to a composition for treating or preventing metastatic foci, comprising the adsorption fraction and a pharmaceutically acceptable carrier.

The present invention relates to a health food for treating or preventing metastatic foci, comprising the adsorption fraction alone or, optionally, with one or more food components.

According to a preferred embodiment of the health food for treating or preventing metastatic foci of the present invention, the health food is a functional food.

The present invention relates to a method for treating or preventing metastatic foci, comprising administering to a subject in need thereof the adsorption fraction in an amount effective therefor.

The present invention relates to use of the adsorption fraction in the manufacture of a composition or health food for treating or preventing metastatic foci.

Further, the present invention relates to an agent for increasing a serum Immunosuppressive Acidic Protein (IAP) value, comprising the adsorption fraction as an active ingredient.

The present invention relates to a composition for increasing a serum IAP value, comprising the adsorption fraction and a pharmaceutically acceptable carrier.

The present invention relates to a health food for increasing a serum IAP value, comprising the adsorption fraction alone or, optionally, with one or more food components.

According to a preferred embodiment of the health food for increasing a serum IAP value of the present invention, the health food is a functional food.

The present invention relates to a method for increasing a serum IAP value, comprising administering to a subject in need thereof the adsorption fraction in an amount effective therefor.

The present invention relates to use of the adsorption fraction in the manufacture of a composition or health food for increasing a serum IAP value.

Further, the present invention relates to an agent for promoting a recovery from stress, comprising the adsorption fraction as an active ingredient.

The present invention relates to a composition for promoting a recovery from stress, comprising the adsorption fraction and a pharmaceutically acceptable carrier.

The present invention relates to a health food for promoting a recovery from stress, comprising the adsorption fraction alone or, optionally, with one or more food components.

According to a preferred embodiment of the health food for promoting a recovery from stress of the present invention, the health food is a functional food.

The present invention relates to a method for promoting a recovery from stress, comprising administering to a subject in need thereof the adsorption fraction in an amount effective therefor.

The present invention relates to use of the adsorption fraction in the manufacture of a composition or health food for promoting a recovery from stress.

Further, the present invention relates to an immune enhancing agent comprising the adsorption fraction as an active ingredient, with the proviso that an agent for treating or preventing metastatic foci and an agent for increasing a serum IAP value are excluded from the immune enhancing'agent.

The present invention relates to an immune enhancing composition comprising the adsorption fraction and a pharmaceutically acceptable carrier, with the proviso that a composition for treating or preventing metastatic foci and a composition for increasing a serum IAP value are excluded from the immune enhancing composition.

The present invention relates to an immune enhancing health food comprising the adsorption fraction alone or, optionally, with one or more food components, with the proviso that a health food for treating or preventing metastatic foci and a health food for increasing a serum IAP value are excluded from the immune enhancing health food.

According to a preferred embodiment of the immune enhancing health food of the present invention, the health food is a functional food.

The present invention relates to a method for an immune enhancement, comprising administering to a subject in need thereof the adsorption fraction in an amount effective therefor, with the proviso that a method for treating or preventing metastatic foci and a method for increasing a serum IAP value are excluded from the method for an immune enhancement.

The present invention relates to use of the adsorption fraction in the manufacture of an immune enhancing composition or health food, with the proviso that a composition or health food for treating or preventing metastatic foci and a composition or health food for increasing a serum IAP value are excluded from the immune enhancing composition or health food.

BRIEF DESCRIPTION OF THE DRAWINGS

Figure 1 is a graph showing a time course for a tumor diameter (mean ± standard deviation) of the right tumor in a double grafted tumor system.

Figure 2 is a graph showing a time course for a tumor diameter (mean ± standard deviation) of the left tumor in a double grafted tumor system.

Figure 3 is a time course for a serum IAP value after administering the M1 fraction or M2 fraction.

Figure 4 is a graph showing that a gene expression of IL-12 in mouse cells from a mesenterium lymph node was induced by orally administering the M2 fraction to mice.

Figure 5 is a graph showing extension of a survival time and effects of anti-IL-12 antibody treatment in mice in which a tumor (P815 tumor cells) was immunized and the M2 fraction was orally administered.

Figure 6 is a graph showing extension of a survival time and effects of anti-IL-12 antibody treatment in mice in which a tumor (colon26 tumor cells) was immunized and the M2 fraction was orally administered

Figure 7 is a spectrum obtained by a $^1$H one-dimensional NMR measurement of the adsorption fraction M2.

Figure 8 is a spectrum obtained by a $^{13}$C one-dimensional NMR measurement of the adsorption fraction M2.

Figure 9 is a broad spectrum obtained by a $^{13}$C one-dimensional NMR measurement of the adsorption fraction M2.

Figure 10 is a CD spectrum obtained by a circular dichroism analysis of the adsorption fraction M2.

Figure 11 is a spectrum obtained by an infrared spectroscopic analysis of the adsorption fraction M2.

Figure 12 is a spectrum obtained by an ultraviolet spectroscopic analysis of the adsorption fraction M2.

Figure 13 is a spectrum obtained by an ESR analysis of the adsorption fraction M2.

Figure 14 is a broad spectrum obtained by an ESR analysis of the adsorption fraction M2.

BEST MODE FOR CARRYING OUT THE INVENTION

The present invention will be explained in detail hereinafter.

The immune enhancing agent or agent for promoting a recovery from stress of the present invention contains as an active ingredient "an adsorption fraction of a liquid mixture by an anion exchange resin", the liquid mixture being obtained by mixing "a hot water extract of a mycelium of *Tricholoma matsutake* FERM BP-7304 [*Tricholoma matsutake (S.Ito* & *Imai) Sing.* CM6271]" (hereinafter sometimes referred to as "hot water extract of the mycelium") with "an alkaline solution extract of a mycelial residue obtained when preparing the hot water extract of the mycelium" (hereinafter sometimes referred to as "alkaline solution extract of the mycelial residue"), wherein

(a) the content of carbohydrates in the adsorption fraction is 60 to 72% (preferably 62 to 70%) as a glucose equivalent determined by a phenol-sulfuric acid method, and
(b) the content of proteins in the adsorption fraction is 28 to 40% (preferably 30 to 38%) as an albumin equivalent determined by a copper-Folin method. Hereinafter, the above "liquid mixture" obtainable by mixing the hot water extract of the mycelium with the alkaline solution extract of the mycelial residue is sometimes referred to as "extract mixture".

The adsorption fraction of the extract mixture by an anion exchange resin, which is used as the active ingredient in the immune enhancing agent or agent for promoting a recovery from stress of the present invention, may be prepared by, for example, but is by no means limited to, a process comprising the steps of:

culturing the *Tricholoma matsutake* FERM BP-7304 strain by a tank culture to obtain the mycelium thereof (hereinafter referred to as a culturing step);
extracting the resulting mycelium with hot water to obtain the hot water extract of the mycelium (hereinafter referred to as a hot water extracting step);
extracting the remaining mycelial residue, after the extraction with hot water, with an alkaline solution to obtain the alkaline solution extract of the mycelial residue (hereinafter referred to as an alkaline solution extracting step);
mixing the hot water extract of the mycelium and the alkaline solution extract of the mycelial residue to obtain the extract mixture, and adsorbing the resulting extract mixture by an anion exchange resin (hereinafter referred to as an anion exchange resin-adsorbing step); and
eluting the'adsorption fraction with an appropriate eluting solution (hereinafter referred to as an eluting step).

The *Tricholoma matsutake* strain FERM BP-7304 (International Application PCT/JP01/08876) used in the culturing step was deposited in the International Patent Organism Depositary National Institute of Advanced Industrial Science and Technology [(Former Name) National Institute of Bioscience and Human-Technology Agency of Industrial Science and Technology (Address: AIST Tsukuba Central 6, 1-1, Higashi 1-chome Tukuba-shi, Ibaraki-ken 305-8566 Japan)] on September 14, 2000. The *Tricholoma matsutake* strain FERM BP-7304 was established as a strain by culturing a piece of fruit body from a *Tricholoma matsutake* strain CM6271 collected in Kameoka-shi, Kyoto, Japan and then culturing it in vitro, and is maintained in the Biomedical Research Laboratories, Kureha Chemical Industry Co. Ltd.
The macroscopical observation of a fruit body of the *Tricholoma matsutake* strain FERM BP-7304 is identical with that of a *Tricholoma matsutake* fruit body described in Rokuya Imaseki and Tsuguo Hongo, "Gensyoku Nihon Shinkinrui Zukan (Colored Illustrations of Mushrooms of Japan) (I)", Hoiku-sha (Osaka, Japan), 1957, plate 9 and page 26. The *Tricholoma matsutake* strain FERM BP-7304 can be subcultivated or maintained on a MATSUTAKE medium. A large-scale cultivation of mycelia of the *Tricholoma matsutake* strain FERM BP-7304 can be carried out by inoculating the strain into a liquid medium and then performing, for example, a static culture, a shake culture, or a tank culture.
When mycelia of the *Tricholoma matsutake* strain FERM BP-7304 are inoculated on a MATSUTAKE medium, white hyphae grow radially and thickly, and form large colonies. According to an observation by a scanning electron microscope, a large number of branched mycelia having a diameter of 1 to 2 $\mu$m exist and projections having a height of approximately a few $\mu$m are observed at the side of mycelia. In this connection, the *Tricholoma matsutake* strain FERM BP-7304 can be subcultured or cultured, generally in the form of mycelia, but sometimes in the form of fruit bodies.
Mycological features of the *Tricholoma matsutake* strain FERM BP-7304 will be explained hereinafter.

(1) Cultural and morphological features on malt extract agar medium

When the *Tricholoma matsutake* strain FERM BP-7304 is inoculated on a malt extract agar medium, white hyphae grow radially and thickly, and form colonies. A diameter of the colony after 30 days from the inoculation is approximately 4 cm.

(2) Cultural and morphological features on Czapek's agar medium, oatmeal agar medium, synthetic mucor agar medium, and medium for assaying phenol oxidase reaction

When the *Tricholoma matsutake* strain FERM BP-7304 is inoculated on a Czapek's agar medium, an oatmeal agar medium, a synthetic mucor agar medium, or a medium for assaying a phenol oxidase reaction, a growth of hyphae is not observed after a month from the inoculation.

(3) Cultural and morphological features on YpSs agar medium

The *Tricholoma matsutake* strain FERM BP-7304 grows as a mat-like mycelia having a white gloss on a YpSs agar medium. A growth area after 30 days from the inoculation is approximately 5 mm in radius.

(4) Cultural and morphological features on glucose and dry yeast agar medium

The *Tricholoma matsutake* strain FERM BP-7304 grows as a mat-like mycelia having white gloss on a glucose and dry yeast agar medium. A growth distance after 30 days from the inoculation is approximately 2 mm.

(5) Optimal growth temperature and range of temperature

After 100 mL-conical flasks each containing 10 mL of sterile medium (3% glucose and 0.3% yeast extract, pH 7.0) were inoculated with approximately 2 mg of a piece of the *Tricholoma matsutake* strain FERM BP-7304 mycelia of the present invention, cultivation was performed at various temperatures from 5 to 35 °C. After cultivation for 28 days, mycelia were taken from the flasks, washed thoroughly with distilled water, and dried, and then each weight of mycelia was measured. As a result, it was found that the weight of mycelia increased linearly at a range of 5 to 15 °C, and gently at a range of 15 to 25 °C. Mycelia did not grow at 27.5 °C or more. The optimal growth temperature is 15 to 25 °C.

(6) Optimal growth pH and range of pH

A growth pH value was examined by preparing various media having a pH in a range of 3.0 to 8.0. These media were prepared by adjusting a pH of a liquid medium (3% glucose and 0.3% yeast extract) with 1 mol/L HCL or 1 mol/L potassium hydroxide. Each medium was sterilized through a filter, and then 10 mL of the sterile medium was poured into a 100 mL-conical flask (previously sterilized). After approximately 2 mg of a piece of the *Tricholoma matsutake* strain FERM BP-7304 mycelia was inoculated, cultivation was performed at 22 °C. After cultivation for 28 days, mycelia were taken from the flasks, washed thoroughly with distilled water, and dried, and then each weight of mycelia was measured. As a result, the limit for growth of mycelia was in a range of a pH 3.0 to 7.0. The optimal growth pH was 4.0 to 6.0.

(7) Formation of confront line by confrontation culture

A block (approximately 3 mm x 3 mm x 3 mm) of the *Tricholoma matsutake* strain FERM BP-7304 and each block (approximately 3 mm × 3 mm × 3 mm) of 13 kinds of known *Tricholoma matsutake* strains were inoculated with a space of approximately 2 cm therebetween on a MATSUTAKE medium. After cultivation was carried out at 22 °C for 3 weeks, it was observed whether or not a zone appeared at the boundary between two colonies.
As a result, it was found that the *Tricholoma matsutake* strain FERM BP-7304 did not form a clear zone with respect to the 13 kinds of known *Tricholoma matsutake* strains. In this connection, it is considered that *Tricholoma matsutake* does not form a zone in a confrontation culture. Among the 13 kinds of known *Tricholoma matsutake* strains, no combinations formed a clear zone.

(8) Auxotrophy

After a 100 mL-conical flask containing 10 mL of a sterile synthetic medium for mycorrhizae (Ohta et al., Trans. Mycol. Soc. Jpn., 31, 323, 1990) was inoculated with approximately 2 mg of a piece of the *Tricholoma matsutake* strain FERM BP-7304 mycelia of the present invention, cultivation was performed at 22 °C. After cultivation for 42 days, the mycelia were taken from the flask, washed thoroughly with distilled water, and dried, and then the weight of mycelia was measured to obtain 441 mg of mycelia.
Each medium in which one of 28 kinds of sugar-related substances was substituted for glucose as a carbon (C) source in the synthetic medium for mycorrhizae was inoculated with the *Tricholoma matsutake* strain FERM BP-7304 of the present invention, and cultivation was performed. After the cultivation, each weight of mycelia was measured.
As a result, an order of the sugar-related substances from that which achieved the heaviest weight to that which achieved the lightest weight was as follows:

wheat starch > corn starch > dextrin > methyl-β-glucoside > cellobiose >mannose > fructose > arabinose > sorbitol
> glucose > lactose > glycogen > mannitol > ribose > maltose > trehalose > galactose > raffinose > melibiose > N-acetylglucosamine.

In this connection, mycelia did not grow in each medium containing cellulose, dulcitol, sucrose, xylose, methyl-$\alpha$-glucoside, inulin, inositol, or sorbose.

Further, each medium in which one of 15 kinds of nitrogen-related substances was substituted for ammonium tartrate as a nitrogen (N) source in the synthetic medium for mycorrhizae was inoculated with the *Tricholoma matsutake* strain FERM BP-7304 of the present invention, and cultivation was performed. After the cultivation, each weight of mycelia was measured.

As a result, an order of the nitrogen-related substances from that which achieved the heaviest weight to that which achieved the lightest weight was as follows: corn steep liquor > soybean peptone > milk peptone > ammonium nitrate > ammonium sulfate > ammonium tartrate > ammonium carbonate > asparagine > ammonium phosphate > ammonium chloride > sodium nitrate > meat extract > yeast extract > casamino acids > chlorella > tryptone > potassium nitrate.

Furthermore, each medium in which one component among minerals and vitamins in the above synthetic medium was removed was inoculated with the *Tricholoma matsutake* strain FERN BP-7304 of the present invention, and cultivation was performed. After the cultivation, each weight of mycelia was measured.

As a result, a deficiency of any one of calcium chloride dihydrate, manganese sulfate (II) pentahydrate, zinc sulfate heptahydrate, cobalt sulfate heptahydrate, copper sulphate pentahydrate, nickel sulfate hexahydrate, thiamin hydrochloride, nicotinic acid, folic acid, biotin, pyridoxine hydrochloride, carnitine chloride, adenine sulfate dihydrate, or choline hydrochloride did not affect the weight of mycelia. In contrast, when any one of magnesium sulfate heptahydrate, iron chloride (II), or sodium dihydrogen phosphate was removed, the weight of mycelia was remarkably lowered. From these results, it is considered that magnesium, iron, phosphorus, and potassium are essential for the growth of the *Tricholoma matsutake* strain FERM BP-7304 of the present invention.

(9) Base composition of DNA (GC content)

The GC content of the *Tricholoma matsutake* strain FERM BP-7304 is 49.9%.

(10) DNA patterns generated by a RAPD method

With respect to each DNA pattern generated by a RAPD (random amplified polymorphic DNA) method using any one of six kinds of primers (10mer) for PCR (polymerase chain reaction), the *Tricholoma matsutake* strain FERM BP-7304 was compared with 44 kinds of known *Tricholoma matsutake* strains (for example, IFO 6915 strain; Institute for Fermentation, Osaka). As a result, it was confirmed that DNA patterns of the *Tricholoma matsutake* strain FERM BP-7304 differed from those of 44 kinds of *Tricholoma matsutake* strains.

As a medium used in the culturing step, for example, a medium composed of glucose and yeast extract may be used.

The concentration of glucose contained in the medium is preferably 0.01% to 15%, more preferably 1% to 10%, most preferably 3%. The concentration of yeast extract contained in the medium is preferably 0.01% to 3%, more preferably 0.1% to 0.6%, most preferably 0.3%. The medium is preferably pH 2.5 to 8,. more preferably pH 4 to 7, most preferably pH 6.

The culturing temperature in the culturing step is preferably 10 to 26 °C, more preferably 15 to 25 °C, most preferably 25 °C.

The period for cultivation is preferably 1 to 20 weeks, more preferably 2 to 10 weeks, most preferably 10 weeks.

As a method for separating mycelia from a broth obtained by cultivation, for example, filtration (for example, filtration with a filter cloth) or centrifugation may be used. It is preferred to wash the resulting mycelia with, for example, distilled water, before the next hot water extracting step is carried out. Further, it is preferred to crush, grind, or pulverize mycelia, to enhance the extraction efficiency.

The temperature of hot water used in the hot water extracting step is preferably 60 to 100 °C, more preferably 80 to 98 °C. It is preferred to carry out the extraction with stirring or shaking, so that the extraction efficiency is enhanced. An extracting time may be appropriately determined in accordance with, for example, the form of mycelia (for example, a crushed, ground, or pulverized form, if treated), the temperature of hot water, or with or without stirring or shaking or a treating condition thereof, but is generally 1 to 6 hours, preferably 2 to 3 hours.

After the hot water extraction, a hot water extract of mycelia can be separated from a mycelial residue by an appropriate separating method, such as centrifugation or filtration.

An alkaline solution used in the alkaline solution extracting step may be, for example, but is by no means limited to, an aqueous solution of a hydroxide of an alkaline metal (such as sodium or potassium), particularly sodium hydroxide. The alkaline solution is preferably pH 8 to 13, more preferably pH 9 to 12. The alkaline solution extraction may be carried out preferably at 0 to 30 °C, more preferably at 0 to 25 °C. An extraction time may be appropriately determined in accordance with, for example, the form of a mycelial residue (for example, a crushed, ground, or pulverized form, if treated), the temperature or pH of an alkaline solution, or with or without stirring or shaking or a treating condition thereof, but is generally 30 minutes to 5 hours, preferably 1 to 3 hours.

After the alkaline solution extraction, an alkaline solution extract of the mycelial residue can be separated from a mycelial residue by an appropriate separating method, such as centrifugation or filtration.

It is preferred to neutralize the resulting alkaline solution extract of the mycelial residue, before the next anion exchange resin-adsorbing step is carried out.

The extract mixture obtained by mixing the hot water extract of mycelia prepared in the hot water extracting step with the alkaline solution extract of the mycelial residue prepared in the alkaline solution extracting step may be used, without a further treatment (i.e., together with insolubles), in the next anion exchange resin-adsorbing step. However, it is preferred to remove the insolubes from the extract mixture, or to remove the insolubles followed by a low molecular fraction therefrom, before the next anion exchange resin-adsorbing step is carried out. For example, the extract mixture in which insolubes are contained may be centrifuged to remove the insolubes, and the resulting supernatant may be used in the next anion exchange resin-adsorbing step. Alternatively, the supernatant obtained by centrifuging the extract mixture containing insolubes may be dialyzed to remove a low molecular fraction (preferable a fraction of substances having a molecular weight of 3500 or less), and the resulting dialyzate may be used in the next anion exchange resin-adsorbing step.

As an anion exchange resin which may be used in the anion exchange resin-adsorbing step, a known anion exchange resin, for example, diethylaminoethyl (DEAE) cellulose or triethylaminoethyl (TEAE) cellulose, may be used.

An eluting solution used in the eluting step may be appropriately selected in accordance with an anion exchange resin used in the anion exchange resin-adsorbing step. For example, an aqueous solution of sodium chloride may be used.

A fraction eluted by the eluting step may be used as the active ingredient of the immune enhancing agent of the present invention as it is, i.e., without purification. However, the fraction eluted by the eluting step usually contains salts derived from the eluting solution, and therefore, it is preferable to dialyze the fraction and remove the salts.

The adsorption fraction of the extract mixture by an anion exchange resin, as the active ingredient in the immune enhancing agent or the agent for promoting a recovery from stress according to the present invention, exhibits the following physicochemical properties.

(1) Carbohydrate content: The content of carbohydrates is 60% to 72% (preferably 62% to 70%) in glucose equivalent by a phenol-sulfuric acid method.

(2) Protein content: The content of proteins is 28% to 40% (preferably 30% to 38%) in albumin equivalent by a copper-Folin method.

(3) Carbohydrate composition: Glucose 61 μg/mg, mannose 3.3 μg/mg, and galactose 2.0 μg/mg.

(4) Amino acid composition: Aspartic acid and asparagine 10.35 mol%, threonine 5.83 mol%, serine 6.27 mol%, glutamic acid and glutamine 10.49 mol%, glycine 8.55 mol%, alanine 9.19 mol%, valine 6.88 mol%, 1/2-cystine 0.60 mol%, . methionine 1.49 mol%, isoleucine 5.36 mol%, leucine 9.25 mol%, tyrosine 2.55 mol%, phenylalanine 4.05 mol%, lysine 5.17 mol%, histidine 2.18 mol%, arginine 4.44 mol%, tryptophan 1.82 mol%, and proline 5.54 mol%.

(5) Isoelectric point: The isoelectric point of the main band is around 5.85 by an isoelectric focusing method.

(6) Nuclear magnetic resonance analysis (NMR)

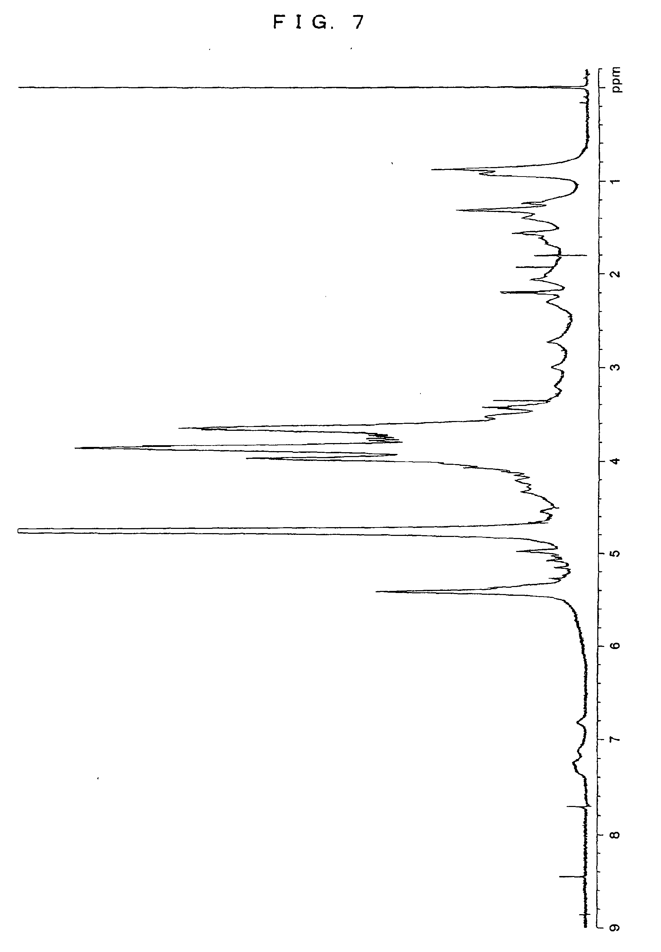

(i) $^1$H one-dimensional NMR analysis: The spectrum is shown in Fig. 7. As to the conditions for measurement, see Example 9(6)(i).

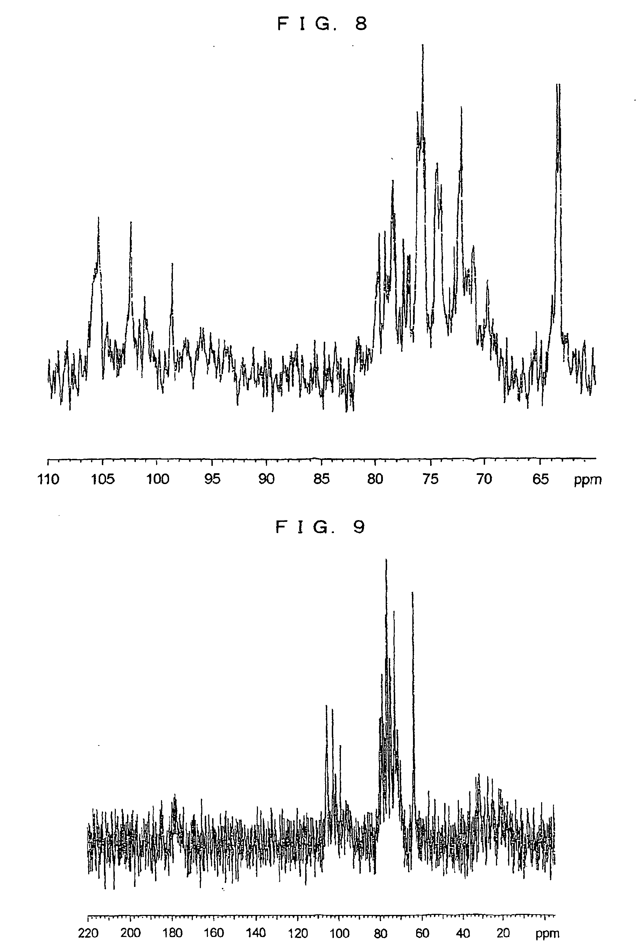

(ii) $^{13}$C one-dimensional NMR analysis: The spectra are shown in Figs. 8 and 9. As to the conditions for measurement, see Example 9(6)(ii).

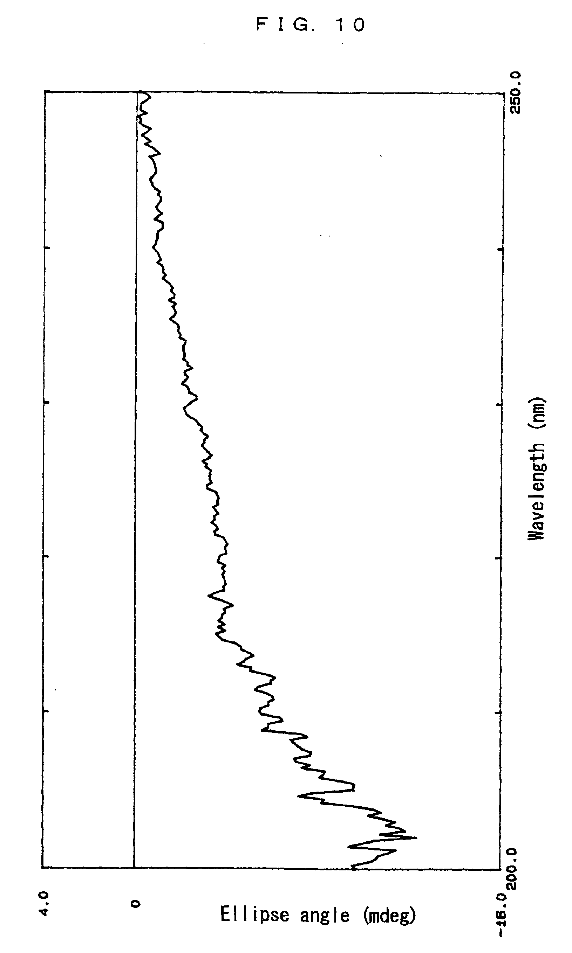

(7) Circular dichroism analysis: The spectrum is shown in Fig. 10. As to the conditions for measurement, see Example 9(7).

(8) Optical rotation: Optical rotation measured at 25 °C is 42.

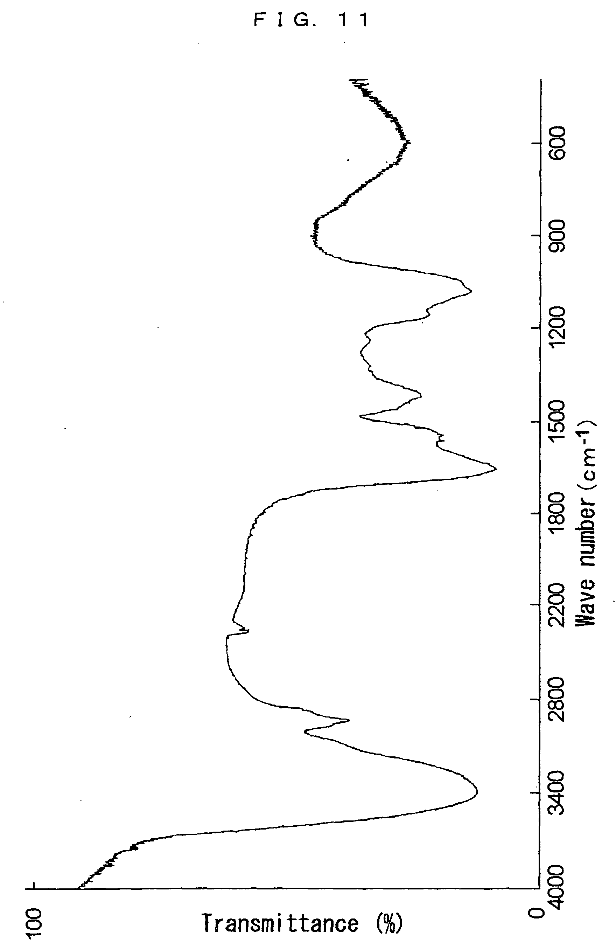

(9) Infrared spectroscopic analysis: The spectrum is shown in Fig. 11. As to the conditions for measurement, see Example 9(9).

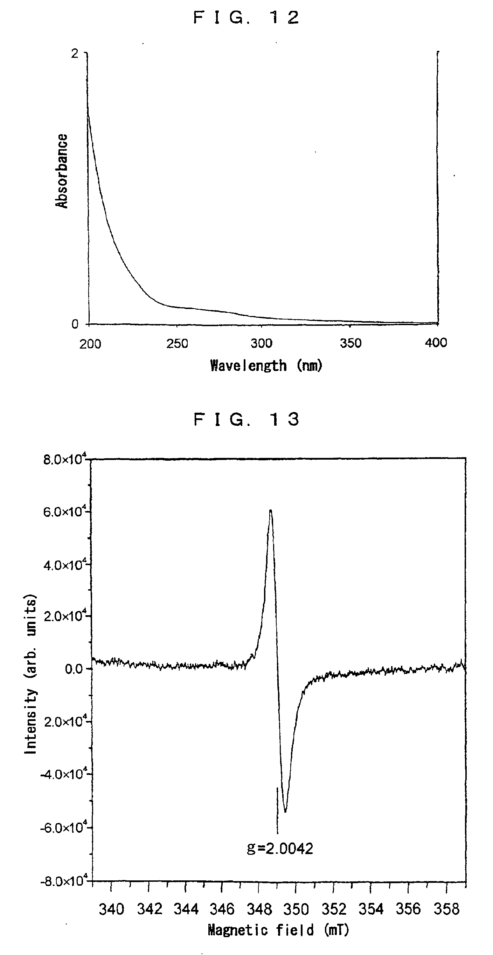

(10) Ultraviolet spectroscopic analysis (UV): The spectrum is shown in Fig. 12. As to the conditions for measurement, see Example 9(10).

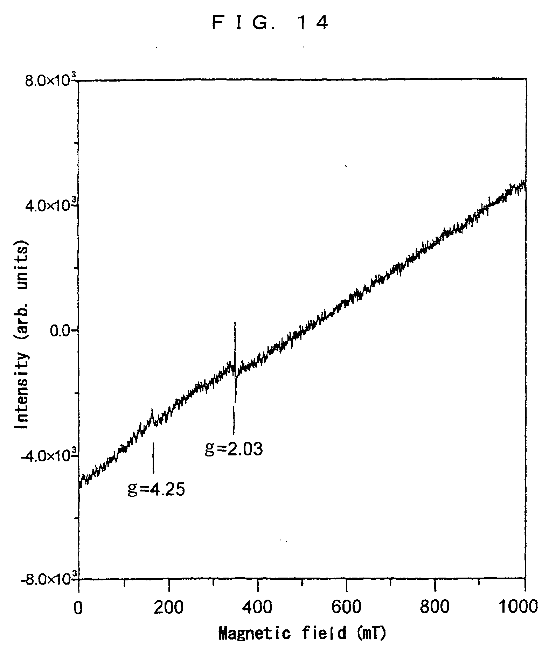

(11) Electron spin resonance (ESR): The spectra are shown in Figs. 13 and 14. As to the conditions for measurement, see Example 9(11).

(12) Viscosity: The reduced viscosity at 30 °C is 108.

(13) Molecular weight: The molecular weight of the major component is 2000 kDa.

(14) Elemental analysis: The contents of carbon (C), hydrogen (H), nitrogen (N), sulfur (S), phosphorus (P), and chlorine (Cl) are 41.3%, 6.0%, 5.1%, 1.0%, 0.052%, and 0.16%, respectively.

(15) Estimated content of $\alpha$-glucan: 71% (with respect to all carbohyderates).

(16) Endotoxin content: 2.5 ng/mg

In the immune enhancing agent or the agent for promoting a recovery from stress according to the present invention, the adsorption fraction of the extract mixture by an anion exchange resin, as the active ingredient, may be administered to an animal (preferably a mammal, particularly a human) with a pharmaceutically or veterinarily acceptable ordinary carrier or diluent. The immune enhancing composition (preferably immune enhancing pharmaceutical composition) or the composition for promoting a recovery from stress (preferably pharmaceutical composition for promoting a recovery from stress) according to the present invention contains the adsorption fraction of the extract mixture by an anion exchange resin, as the active ingredient, and a pharmaceutically or veterinarily acceptable ordinary carrier or diluent.

The active ingredient in the immune enhancing agent of the present invention, i.e., the adsorption fraction of the extract mixture by an anion exchange resin, exhibits an immuno-enhancing activity. The immune-enhancing activity includes, for example, an antitumor activity [for example, an activity to extend a survival time in a subject with cancer, an anti-primary tumor activity (particularly an activity to inhibit a primary tumor proliferation), or an antimetastasis activity (particularly an activity to inhibit a metastatic focus proliferation)], an activity to produce an induction of a killer activity (particularly an activity to produce an induction of a killer activity of an intestinal lymphocyte), an activity to enhance recognition of a tumor cell, an activity to enhance a gene expression of interleukin 12 (IL-12), and an activity to increase a serum IAP value. The immune enhancing agent of the present invention has an antitumor activity, such as an anti-primary tumor activity (particularly an activity to inhibit a primary tumor proliferation) or an antimetastasis activity (particularly an activity to inhibit a metastatic focus proliferation), and thus it may be used for treating or preventing cancer, such as a primary tumor or metastatic foci.

Therefore, the active ingredient in the present invention, i.e., the adsorption fraction of the extract mixture by an anion exchange resin, may be administered alone or, preferably, together with a pharmaceutically or veterinarily acceptable ordinary carrier or diluent to a subject in need of an immune enhancement in an amount effective therefor.

Further, the active ingredient in the present invention, the adsorption fraction of the extract mixture by an anion exchange resin, may be used in the manufacture of an immune enhancing composition (preferably immune enhancing pharmaceutical composition), an immune enhancing health food (preferably immune enhancing functional food), or an oral hygienic composition for an immune enhancement.

The active ingredient in the agent of the present invention for promoting a recovery from stress, i.e., the adsorption fraction of the extract mixture by an anion exchange resin, exhibits an activity of promoting a recovery from stress.

Therefore, the active ingredient in the present invention, the adsorption fraction of the extract mixture by an anion exchange resin, may be administered alone or, preferably, together with a pharmaceutically or veterinarily acceptable ordinary carrier or diluent to a subject in need of promoting a recovery from stress in an amount effective therefor.

Further, the active ingredient in the present invention, the adsorption fraction of the extract mixture by an anion exchange resin, may be used in the manufacture of a composition for promoting a recovery from stress (preferably pharmaceutical composition for promoting a recovery from stress), a health food for promoting a recovery from stress (preferably functional food for promoting a recovery from stress), or an oral hygienic composition for promoting a recovery from stress.

Generally, when stress is loaded to an animal once or during a certain period, an immune activity in the animal is decreased but the immune activity is spontaneously recovered after a release of the stress. The "activity of promoting a recovery from stress" as used herein means an activity which promotes the recovery of an immune activity during a period of an immune activity convalescence after a release of stress, in comparison with the spontaneous recovery.

The agent of the present invention for promoting a recovery from stress can be administered at any time, so long as it can promote the recovery of an immune activity briefly lowered by stress. It can be administered, for example, before a loading of stress, during a loading of stress, and/or during a period of an immune activity convalescence after a release of stress.

In this connection, the "activity of promoting a recovery from stress" in the present invention is different from the above-described mere "immuno-enhancing activity" found by the present inventor. Generally, the "immuno-enhancing activity" means an activity in which an enhancement of an immune activity is observed by administering an active ingredient having such an activity in comparison with a state before the administration, that is, an activity of enhancing an immune activity per se. The state before the administration may be a state in which an immune activity is natural or lowered by stress. In contrast, the "activity of promoting a recovery from stress" in the present invention is an activity

# EP 1 477 179 A1

which promotes the recovery of an immune activity during a period of an immune activity convalescence, as described above, that is, an activity of enhancing the speed of recovery of an immune activity. When the agent of the present invention for promoting a recovery from stress is administered, the speed of recovery of an immune activity is increased in comparison with the case in which the agent of the present invention for promoting a recovery from stress is not administered.

Further, in the "immuno-enhancing activity", enhancement of an immune activity is directly observed when administering an active ingredient having such an activity. In contrast, when administering the active ingredient of the agent of the present invention for promoting a recovery from stress (i.e., the adsorption fraction of the extract mixture by an anion exchange resin) to a subject animal before a loading of stress, the recovery of an immune activity is promoted during a period of an immune activity convalescence, even if the adsorption fraction of the extract mixture by an anion exchange resin is not administered during a loading of stress and during a period of an immune activity convalescence. With respect to the point, the "activity of promoting a recovery from stress" in the present invention is different from the "immuno-enhancing activity" in the present invention.

The formulation of the immune enhancing agent or agent for promoting a recovery from stress of the present invention is not particularly limited to, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants, such as sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous or intravenous injection, or a per rectum administration. Of the parenteral formulations, an injection is preferably used.

When the injections are prepared, for example, watersoluble solvents, such as physiological saline or Ringer's solution, water-insoluble solvents, such as plant oil or fatty acid ester, agents for-rendering isotonic, such as glucose or sodium chloride, solubilizing agents, stabilizing agents, antiseptics, suspending agents, or emulsifying agents may be optionally used, in addition to the active ingredient.

The immune enhancing agent or agent for promoting a recovery from stress of the present invention may be administered in the form of a sustained release preparation using sustained release polymers. For example, the immune enhancing agent of the present invention may be incorporated to a pellet made of ethylenevinyl acetate polymers, and the pellet may be surgically implanted in a tissue to be treated.

The immune enhancing agent or agent for promoting a recovery from stress of the present invention may contain the adsorption fraction of the extract mixture by an anion exchange resin in an amount of, but is by no means limited to, 0.01 to 99% by weight, preferably 0.1 to 80% by weight.

A dose of the immune enhancing agent or agent for promoting a recovery from stress of the present invention is not particularly limited, but may be determined dependent upon the kind of disease, the age, sex, body weight, or symptoms of the subject, a method of administration, or the like. The immune enhancing agent or agent for promoting a recovery from stress of the present invention may be orally or parenterally administered.

The agent of the present invention may be administered as a medicament or in various forms, for example, eatable or drinkable products such as health foods (preferably functional foods) or feeds. The term "foods" as used herein includes drinks.

It is known that food has (1) a function as nutrients (the first function), (2) a function which appeals to five senses in a human (the second function), and (3) a function which affects a favorable influence on health, physical ability, or mental condition in a human (the third function). The third function regulates various physiological systems, such as the digestive system, circulatory system, endocrine system, immune system, or nervous system, and affects a favorable influence on the maintaining or recovery of health. The term "health food" as used herein means food which provides, or is expected to provide, one or more effects on health. The term "functional food" as used herein means processed or designed food such that the above various biological regulatory functions (i.e., functions which regulate physiological systems such as the digestive system, circulatory system, endocrine system, immune system, or nervous system) may be fully expressed.

Further, the agent of the present invention may be administered in the form of an oral hygienic composition which is temporarily kept in the mouth but then spat out, retaining almost none of the components, for example, a dentifrice, a mouthwash agent, a chewing gum, or a collutorium, or in the form of an inhalation drawn in through the nose. More particularly, the adsorption fraction of the extract mixture by an anion exchange resin may be added to a desired food including a drink, a feed, a dentifrice, a mouthwash agent, a chewing gum, a collutorium, or the like as an additive, such as a food additive.

**10**

EXAMPLES

The present invention now will be further illustrated by, but is by no means limited to, the following Examples.

Example 1: Preparation of an adsorption fraction of an extract mixture from mycelia of *Tricholoma matsutake* FERM BP-7304 by an anion exchange resin

After a 7 t-culture tank containing 3.5 t of sterile medium (3% glucose and 0.3% yeast extract, pH 6.0) was inoculated with mycelia of *Tricholoma matsutake* FERM BP-7304, cultivation was performed with shaking at 25 °C for 4 weeks. The resulting broth was filtered with a filter cloth to separate mycelia. The mycelia were washed with distilled water.

After 30 L of purified water was added to a portion (about 1 kg) of the resulting mycelia, an extraction treatment was performed in a water bath at 98 °C for 3 hours while stirring. The whole was cooled and centrifuged at 8000 rpm for 30 minutes to obtain a supernatant $A_1$. After 30 L of purified water was added to the remaining pellets, extraction and centrifugation were performed in a similar manner as described above to obtain a supernatant $A_2$.

After 20 L of 0.5 mol/L sodium hydroxide solution was added to the remaining pellets, extraction was carried out at 25 °C for 1 hour, and the whole was centrifuged to obtain a supernatant $B_1$. After 1.0 mol/L sodium hydroxide solution was added to the remaining pellets, extraction and centrifugation were performed in a similar manner to obtain a supernatant $B_2$. The resulting supernatants $B_1$ and $B_2$ were combined and adjusted to pH 7.0 by adding 1.0 mol/L HCl (hereinafter referred to as supernatant B).

After the supernatants $A_1$, $A_2$, and B were combined, the resulting mixture (hereinafter referred to as extract mixture M) was put into a dialysis tube (fractioning molecular weight = 3500), and dialyzed in flowing water for 48 hours. The inner part of the dialyzate was collected and dried by a lyophilizer to obtain approximately 70 g of white dry powder.

A portion (10 g) of the resulting powder was dissolved in 500 mL of 50 mmol/L tris-HCl buffer (pH 7.0), and the solution was applied to a diethylaminoethyl (DEAE) sephacel (Pharmacia) column which had been equilibrated with the same buffer to obtain a fraction which passed through the column, as a non-adsorption fraction M1. After the column was washed with the tris-HCl buffer, a 50 mmol/L tris-HCl buffer (pH 7.0) containing 0.5 mol/L of sodium chloride was applied to the column to obtain an eluted fraction, as an adsorption fraction M2.

The resulting M1 fraction and M2 fraction were dialyzed in distilled water for injection at 4 °C for 48 hours. The inner part of the dialyzate was lyophilized to obtain powder. The yields (with respect to a dry weight of mycelia) of the M1 and M2 fractions were 7% and 13%, respectively.

Example 2: Evaluation of the adsorption fraction M2 in double grafted tumor system

In the present example, an immune-enhancing activity of the M2 fraction prepared in Example 1 was evaluated by a double grafted tumor system as an artificial model for a metastasis.

More particularly, 7-week-old male BALB/c mice (one group consisting of 7 mice; Japan SLC) and syngeneic Meth-A fibrosarcomas were used. Each mouse received simultaneous intradermal inoculations of Meth-A cells in the right ($2 \times 10^6$) and left ($4 \times 10^5$) flanks. After three days from the inoculation [a tumor in the right flask (regarded as a primary focus) became large so that the tumor could be handled by the fingers], 5 mg of the M1 or M2 fraction prepared in Example 1 was repetitively administered into the right tumor for 3 days, and the growth of the left tumor (regarded as a metastatic focus) and the right tumor was observed for 21 days. Instead of the M1 or M2 fraction, physiological saline was administered to a control group.

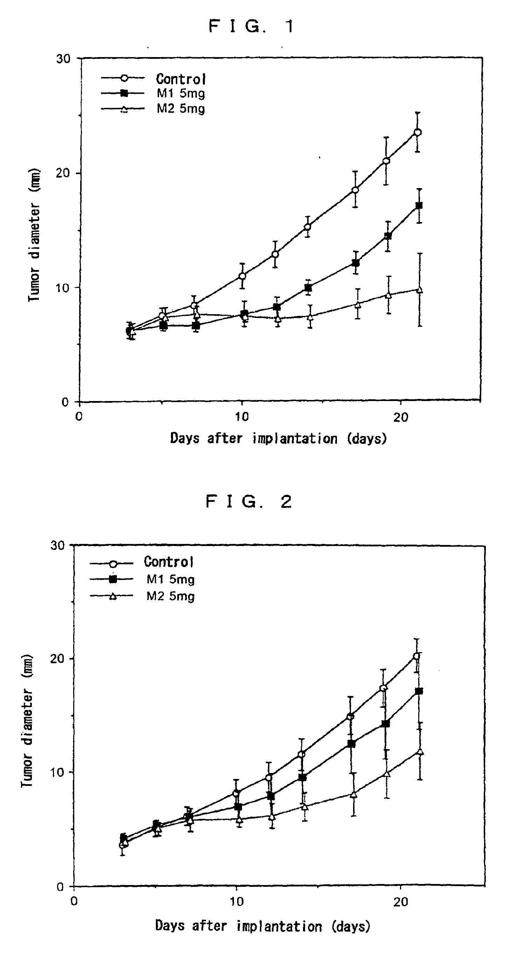

The results are shown in Figs. 1 and 2 and Table 1.

Fig. 1 is a graph showing a time course for a tumor diameter (mean ± standard deviation) of the right tumor. Fig. 2 is a graph showing a time course for a tumor diameter (mean ± standard deviation) of the left tumor. In the Figs. 1 and 2, white circles represent the results in the control group, black squares represent those for the M1 fraction, and white triangles represent those for the M2 fraction.

Table 1 shows each tumor diameter (mean ± standard deviation; unit = mm) and tumor weight (mean ± standard deviation; unit = g) of the right and left tumors after 21 days from the inoculation.

Administration of the M2 fraction significantly inhibited the growth of the left and right tumors, in comparison with the control group. The effect was remarkable in the right tumor to which the M2 fraction was directly injected. The growth of the left tumor without injection was also inhibited, and thus it was considered that the effect in the left tumor was caused by a mechanism via immunity (cytokines).

Table 1

| | Right tumor ($2\times10^6$ cells) | | Left tumor ($4\times10^5$ cells) | |
|---|---|---|---|---|
| | Diameter (mm ± S. D.) | Weight (g ± S. D.) | Diameter (mm ± S. D.) | Weight (g ± S. D.) |
| Control | 23.5 ± 1.7 | 4.7 ± 1.0 | 20.3 ± 1.5 | 3.0 ± 1.0 |
| M1 | 17.1 ± 1.5 | 1.8 ± 0.5 | 17.1 ± 3.4 | 2.1 ± 1.1 |
| M2 | 9.7 ± 3.2 | 0.6 ± 0.4 | 11.8 ± 2.5 | 0.7 ± 0.5 |

Example 3: Measurement of serum IAP level

In the present example, a change of serum IAP (immunosuppressive acidic protein) levels by administration of the M2 fraction was observed. It is known that the serum IAP is produced by activated macrophages, and thus the serum IAP level may be used as an indicator of a macrophage activation.

More particularly, 5 mg of the M1 or M2 fraction prepared in Example 1 was intradermally injected to BALB/ c mice, blood was collected with time, and the serum IAP levels therein were measured by a SRID (single radial immunodiffusion) method (J. Clausen, translated by Sai Sasaki and Takashi Murachi, "Immunological identification methods", Tokyo Kagaku Dojin, 18-21, 1973).

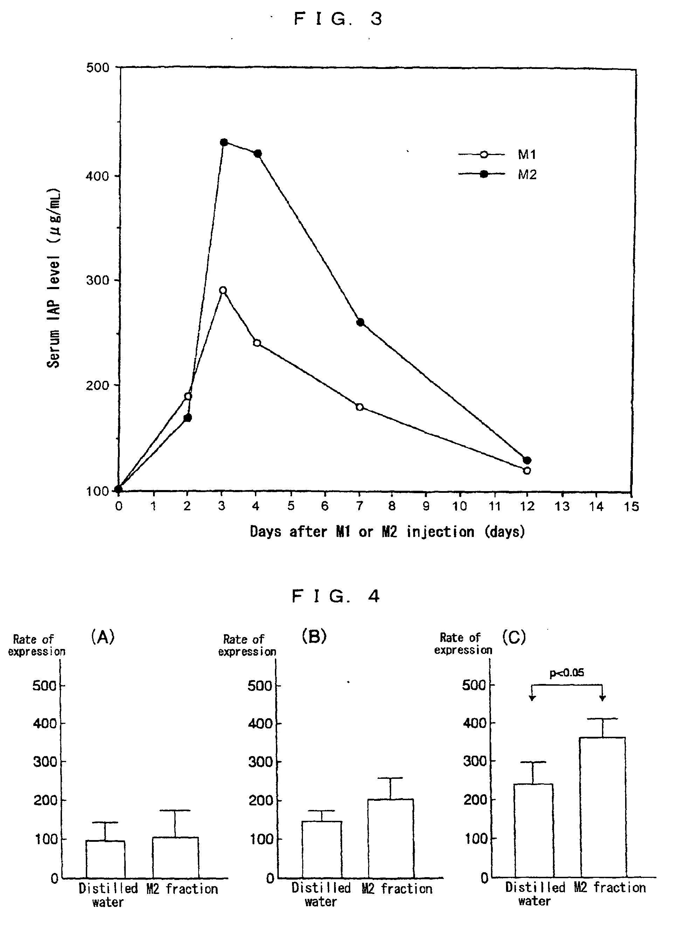

The results are shown in Fig. 3. In Fig. 3, white circles represent the results for the M1 fraction, and black circles represent the results for the M2 fraction.

As shown in Fig. 3, 430 µg/mL of IAP was produced by administration of the M2 fraction.

Example 4: Evaluation for activity of the adsorption fraction M2 for inhibiting tumor (sarcoma 180) proliferation

As an animal subject, female ICR mice (CLEA Japan, Inc.) were used. As a tumor, sarcoma 180 cells maintained in the peritonium of a female ICR mouse in Biomedical Research Laboratories, Kureha Chemical Industry Co. Ltd., were used. More particularly, sarcoma 180 cells ($1\times10^6$) were transplanted at an axilla of 5-week-old female ICR mice (one group consisting of 10 mice). From the day after the transplanting, a predetermined amount (1.0 mg/kg, 10 mg/kg, or 50 mg/kg) of the adsorption fraction M2 obtained in Example 1 was intraperitoneally administered every other day, for 10 times in total. On the 25th day after the transplantation, mice were sacrificed, tumor nodes were taken, and the weights were measured. Physiological saline was administered to the mice of the control group.

The ratio of proliferation inhibition (unit = %) was calculated by the following equation:

$$[\text{Ratio of proliferation inhibition (\%)}] = \{(Wc-W)/Wc\}\times100$$

wherein W is an average weight (unit = g) of the node taken from the group treated with the sample, and Wc is an average weight (unit = g) of the node taken from the group treated with the physiological saline.

The results are shown in Table 2. As apparent from Table 2, proliferation was significantly inhibited by administering the M2 fraction.

Table 2

| No. | Sample/Dose | Ratio of proliferation inhibition (%) |
|---|---|---|
| 1 | M2 fraction/1.0 mg/kg | 71% |
| 2 | M2 fraction/10 mg/kg | 81% |
| 3 | M2 fraction/50 mg/kg | 48% |

Example 5: Evaluation of the adsorption fraction M2 for influence producing induction of killer activity of intestinal lymphocytes

The M1 fraction, the M2 fraction, and the extract before fractionation thereof (i.e., lyophilized powder of the extract mixture M), prepared in Example 1, were orally administered to mice, and an influence producing an induction of a killer activity of intestinal lymphocytes was examined. The activity of cells from a mesenterium lymph node was evaluated by a method of Harada et al. (Harada M. et al., Cancer Res., 55, 6146-6151, 1995).

More particularly, biological activities were examined by taking cells of a mesenterium lymph node from a mouse to which tumor cells had been implanted at a cecal wall, and measuring a killer activity obtained when the tumor cells were re-stimulated in a test tube. The tumor cells used in this Example were mouse leukemic cells P815 and B7/P815 which were originally supplied from Dr. Mamoru Harada, Medical Institute of Bioregulation, Kyushu University (currently, Department of Medicine, Kurume University), and maintained in an RPMI 1640 medium containing 10% bovine fetal serum which had been heated at 56 °C for 30 minutes, at Biomedical Research Laboratories, Kureha Chemical Industry Co. Ltd. Female DBA/2 mice were purchased from Japan SLC and used in experiments at 8 weeks-old after pre-breeding.

The mice were anesthetized by intraperitoneally administering 50 mg/kg of Nembutal and fixed. An abdomen was opened by scissors and tweezers, a cecum taken out, B7/P815 cells (1 x $10^6$/50 µL) were implanted at a cecal wall, using 1 mL syringe equipped with a 1/8G dental needle (Hatajirushi Motoki Syringe Needle), and then the abdomen was closed by anatomical staplers. The mice which had recovered from anesthetization were put into a breeding cage, and bred under ordinary breeding conditions. From the day after the implantation of the tumor cells, each sample was orally administered for 10 days in succession, using a probe for an oral administration. A group of mice contained 10 mice.

On the day after the last administration day, the mice were sacrificed. Then, a lymph node of a mesenterium was aseptically taken out, put on a sterilized dish containing a Hanks balanced salt solution, teased by scissors and tweezers, and passed through a mesh to prepare a single-cell suspension of lymphocyte cells. The cells were washed with an RPMI 1640 medium containing a 10% bovine fetal serum which had been heated at 56 °C for 30 minutes three times. Then, a concentration of cells was adjusted to 5 x $10^6$/mL with an RPMI 1640 medium containing a 10% bovine fetal serum which had been heated at 56 °C for 30 minutes, 5 x $10^{-5}$ mol/L of 2-mercaptoethanol, 20 mmol/L of 4-(2-hydroxyethyl)-1-piperazine ethanesulfonate, and 30 µg/mL of gentamicin and used as effector cells.

Stimulating cells were prepared as follows: P815 cells or B7/P815 cells were suspended in an RPMI 1640 medium, so that a concentration became 5 x $10^6$/mL, and mitomycin C (Sigma) was added thereto so that a concentration thereof was 50 µg/mL. After a reaction was performed in a 5% carbon dioxide gas incubator for 30 minutes, the cells were washed with an RPMI 1640 medium containing a 10% bovine fetal serum which had been heated at 56 °C for 30 minutes three times, and a concentration of cells was adjusted to 1 x $10^5$/mL.

A mixed lymphocyte tumor cell reaction (MLR) was examined under the following conditions.

The effector cells (0.1 mL) and/or the stimulating cells (0.1 mL) were put on a 96-well culturing flat-bottomed microplate (Falcon 3072; Becton Dickinson Labware, USA), cultured in a 5% carbon dioxide gas incubator at 37 °C for 3 days, and recovered on a filter. When both the effector cells and the stimulating cells were put into the microplate, a ratio of the cells (the number of the effector cells/the number of the stimulating cells) was 12.5. In this examination system, the effector cells functioned as lymphocytes in the mixed lymphocyte tumor cell reaction, and the stimulating cells functioned as tumor cells in the mixed lymphocyte tumor cell reaction. Before 8 hours of completing the culturing, 37 kBq of $^3$H-thymidine (Amersham Japan) was added to each well of the plate. The harvested cells were thoroughly washed with 5% trichloroacetic acid, dried, and put into a vial for liquid. After adding a liquid scintillator, the radioactivity was measured by a liquid scintillation counter.

A stimulation index (S.I.) was calculated by an equation:

$$[S.I.]=(Bmix-Bs)/(Be-Bs)$$

wherein Bmix is a radioactivity (unit = Bq) of a group of the mixed culture of the effector cells and the stimulating cells, Bs is a radioactivity (unit = Bq) of a group of the single culture of the stimulating cells, and Be is a radioactivity (unit = Bq) of a group of the single culture of the effector cells.

An MLR-cell-mediated Cytotoxicity (MLR-CMC) was examined under the following conditions.

The effector cells (1.0 mL) and the stimulating cells (1.0 mL) were put on a 24-well culturing microplate (Culture Clastar; Costar 3524; Corning Inc., USA) at a ratio of the cells (the number of the effector cells/the number of the stimulating cells) of 12.5, and cultured in a 5% carbon dioxide gas incubator for 3 days. After the culturing was completed, the cells were recovered and washed three times with an RPMI 1640 medium containing 10% bovine fetal serum which had been heated at 56 °C for 30 minutes, and the number of only the effector cells in the cell suspension was counted, using a microscope, so that a concentration of the effector cells was adjusted to 2.5 x $10^6$/mL.

P815 cells were reacted with sodium chromate (Amersham Japan) at 37 °C for 20 minutes. Unreacted radioactive substances were removed by washing with an RPMI 1640 medium containing 10% bovine fetal serum which had been heated three times at 56 °C for 30 minutes, and a concentration of tumor cells labeled with radioactive chromium was adjusted to 5 x $10^4$/mL.

0.1 mL of the effector cells or a double-diluted series thereof and 0.1 mL of tumor cells labeled with radioactive chromium were put into a test tube, and reacted in a 5% carbon dioxide gas incubator at 37 °C for 4 hours. After the reaction was completed, 1.5 mL of an RPMI 1640 medium containing a 10% bovine fetal serum, which had been heated at 56 °C for 30 minutes, was added to each test tube, and thoroughly mixed by a mixer. The whole was centrifuged at 12,000 rpm for 5 minutes at 4 °C to obtain a supernatant, and a radioactivity was measured by a gamma counter.

A specific lysis (S.L.; unit = %) was calculated by an equation:

$$[S.L.(\%)]=\{(B-Bf)/(Bmax-Bf)\} \times 100$$

wherein B is a radioactivity (unit = Bq) of a supernatant of an experimental group, Bf is a radioactivity (unit = Bq) of a supernatant of a spontaneously releasing group, and Bmax is a radioactivity (unit = Bq) of a supernatant of a maximum releasing group. The spontaneously releasing group means a group of culturing only the tumor cells labeled with radioactive chromium, and the maximum releasing group means a group of culturing tumor cells labeled with radioactive chromium treated with 5% Triton X100.

The results of the influence on the MLR-CMC for the samples prepared in Example 1 are shown in Table 3, when the ratio of the number of the effector cells/the number of the stimulating cells was 12.5. In Table 3, "*" means that there was a significant difference of p<0.01 on the basis of the control group. To control groups, 0.2 mL of purified water was orally administered. As apparent from Table 3, the M2 fraction had the activity for inducing the MLR-CMC, and the activity exhibited a dose-dependency.

Table 3

| Experimental No. | Sample/Dose | MLR-CMC (% to the control group) |
|---|---|---|
| 1 | Extract(before fractionation)/250 mg/kg | 153* |
| | M1/250 mg/kg | 109 |
| | M2/250 mg/kg | 181* |
| 2 | M2/ 63 mg/kg | 131* |
| | M2/125 mg/kg | 159* |
| | M2/250 mg/kg | 197* |

Example 6: Evaluation of the adsorption fraction M2 for activity to enhance gene expression of IL-12

The gene expression of interleukin 12 (IL-12) in cells from a mesenterium lymph node was measured by a reverse transcriptase-polymerase chain reaction (RT-PCR) method in accordance with a method of Harada et al. (Harada M. et al., Cancer Res., 58, 3073-3077, 1998).

More particularly, tumor cells were implanted in mice at a cecal wall thereof in a manner similar to that in Example 5. Instead of tumor cells, physiological saline was injected to a cecal wall to prepare sham mice. To the tumor-implanted mice, the sham mice, and normal mice, 250 mg/kg/day of the M2 fraction or distilled water was administered. After 11 days from the implantation, mice were sacrificed, and then mesenterium.lymph nodes were taken, immediately frozen in liquid nitrogen, and kept at -80 °C until analysis.

The following procedures were carried out in accordance with protocols attached to commercially available kits or reagents, unless otherwise specified. Total RNA was prepared from each sample by a reagent for RNA preparation (TRIzol reagent; Life Technologies, Inc., USA). The resulting total RNAs were reverse-transcribed using a reverse transcriptase (Superscript reverse transcriptase; Life Technologies, Inc.), random hexamers, and deoxyribonucleoside triphosphates, to synthesize cDNAs. The cDNAs were amplified in the presence of Taq DNA polymerase (Perkin-Elmer Co.,USA), deoxyribonucleoside triphosphates, and a set of primers, using a PCR thermal cycler (Perkin-Elmer PCR Thermal Cycler TP-2000; Perkin-Elmer Co.).

In the above PCR, cycle numbers in the case of IL-12 p40 and β-actin were 30 and 36, respectively. As to the primer set, oligonucleotides consisting of the base sequences of SEQ ID NOS: 1 and 2 were used as sense and antisense primers for IL-12 p40. Oligonucleotides consisting of the base sequences of SEQ ID NOS: 3 and 4 were used as sense and antisense primers for β-actin.

The PCR products were electrophoresed using an agarose gel containing ethidium bromide, and digitized using a multi-analyst system (Bio-Rad Multi-Analyst System).

The results are shown in Fig. 4. In Fig. 4, graph (A) shows the results in normal mice, graph (B) shows the results in sham mice, and graph (C) shows the results in tumor-implanted mice. In Fig. 4, a rate of the IL-12 p40 gene expression is represented by a percentage with respect to the normal and distilled water-administered mice group.

The IL-12 gene expression was enhanced in the tumor-implanted and distilled water-administered mice group, in comparison with the normal and distilled water-administered mice group. The M2 fraction little affected the IL-12 gene expression in the normal mice, but significantly promoted it in the tumor-implanted mice. Further, the M2 fraction promoted it slightly in the sham mice.

Example 7: Evaluation for extension of survival time and effect of anit-IL-12 antibody treatment in mice in which tumor was immunized and the M2 adsorption fraction was orally administered

In the present example, an influence of the M2 fraction on an antitumor activity was examined. When B7/P815 cells were implanted in a mouse cecum, a tumor proliferated at first, but tumor regression occurred after 14 days from the implantation, and all mice survived. By contrast, when P815 cells were implanted at a mouse cecal wall, tumor cells proliferated and metastasized, and all mice died of tumor. Therefore, B7/P815 tumor cells ($5 \times 10^5$ cells/dose) were immunized in a cecum of DBA/2 mice three times at intervals of two weeks, and after two weeks from the last injection, P815 cells ($1 \times 10^5$ cells) were implanted in a cecum thereof, and the survival time of each mouse was observed. In this connection, with respect to the proliferation of P815 tumor cells in a cecum, the group in which B7/P815 cells were immunized in a cecum was superior to the subcutaneously-immunized group.

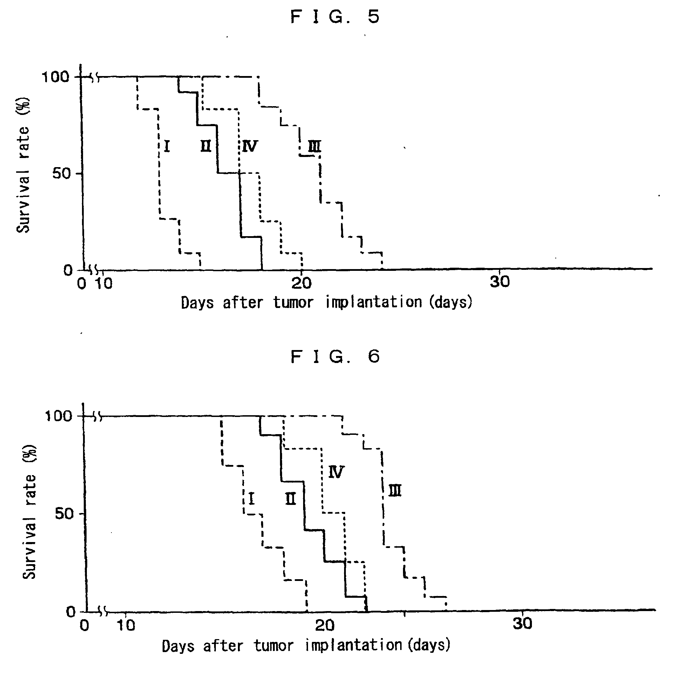

The results are shown in Table 4 and Fig. 5. In Table 4 and Fig. 5, the group I is a nonimmunized and distilled water-administered group; the group II is an immunized and distilled water-administered group; the group III is an immunized and M2-administered group in which the M2 fraction (250 mg/kg) was orally administered every day during the immunized period; and the group IV is an immunized, M2-administered, and anti-IL-12 antibody-treated group in which the M2 fraction (250 mg/kg) was orally administered every day during the immunized period and an anti-IL-12 antibody (200 μg/dose) was intravenously injected before 12 days, 7 days, and 2 days from the implantation of P815 cells. In Table 4, "SD" means a standard deviation, "T" in "T/C" means a survival time in the test groups (i.e., groups II to IV), "C" means a survival time in the control group (i.e., group I), and "*" means that there was a significant difference of $p<0.01$ with respect to the group II.

Table 4

| Group | Survival time mean ± SD | (T/C) ×100 |
|---|---|---|
| I | 13.2 ± 0.8 | - |
| II | 16.3 ± 1.2 | 100 |
| III | 20.8 ± 1.9 | 128* |
| IV | 17.7 ± 1.2 | 109 |

As shown in Fig. 5, the survival time was extended by approximately 120% in the immunized and distilled water-administered group (group II), in comparison with the nonimmunized and distilled water-administered group (group I). The survival time was significantly extended by 128% in the immunized and M2-administered group (group III), in comparison with the immunized and distilled water-administered group (group II). In the immunized, M2-administered, and anti-IL-12 antibody-treated group (group IV), the survival time was attenuated to 109% of that in the immunized and distilled water-administered group (group II).

In a similar fashion, colon26 tumor cells treated with mitomycin C were immunized to BALB/c mice at a cecal wall three times at intervals of two weeks, and colon26 living cells were implanted. The results are shown in Table 5 and Fig. 6. The groups I to IV in Table 5 and Fig. 6 have the same meanings in Table 4 and Fig. 5.

Table 5

| Group | Survival time mean ± SD | (T/C) ×100 |
|---|---|---|
| I | 16.8 ± 1.5 | - |
| II | 19.4 ± 1.6 | 100 |
| III | 23.3 ± 1.3 | 120* |
| IV | 20.4 ± 1.4 | 105 |

In the nonimmunized and distilled water-administered , group (group I), almost all of the mice died of cachexia, intestinal obstruction caused by tumor proliferation, or peritonitis. However, oral administration of the M2 fraction significantly extended the survival time of the mice. The effect was attenuated by the treatment of anti-IL-12 antibody. The administration of the M2 fraction to the nonimmunized mice did not affect the survival time.

Example 8: Evaluation of the adsorption fraction M2 for activity of promoting a recovery from stress

In the present example, the M2 fraction prepared in Example 1 was used as a sample for evaluation. Further, after the sample for evaluation was orally administered to mice for 10 days, restraint stress was loaded for 18 hours, and then a natural killer (NK) cell activity was measured after a release of the stress to examine the effects of the sample.

More particularly, an aqueous solution of the sample for evaluation was orally administered to 8-week-old male C57BL/6 mice (purchased from Charles River Japan; 5 to 10 mice per a group) at a dose of 300 mg/kg/day for 10 days in normal breeding cages. After the administration for 10 days, mice were transferred from the normal breeding cages to 50 mL-capped polypropylene centrifuge tubes (catalog No. 2341-050; Asahi Techno Glass Corporation) with air vents so that a mouse was confined in a tube, The confined mice could not move in the tubes. The tubes in which mice were confined were placed in the cages and allowed to stand for 18 hours to load the mice with restraint stress. After the stress loading for 18 hours, mice were transferred from the tubes to the breeding cages, and bred under ordinary breeding conditions.

After 7 days had passed from the release of the restraint stress, mice were sacrificed, and a natural killer (NK) cell activity was evaluated by measuring a cytotoxic activity of lymphocytes against an NK-sensitive tumor cell strain YAC-1 in vitro, in accordance with a method of Greenberg et al. (Greenberg A.H. et al., J.Exp.Psychol., 12, 25-31, 1986) as follows.

Spleens and mesenterium lymph nodes were aseptically taken from mice and transferred to a sterile petri dish containing a Hanks balanced salt solution. The lymph nodes were teased with scissors and tweezers, and passed through a mesh to prepare a suspension containing single lymphocyte cells. The cells were washed three times with an RPMI 1640 medium containing a 10% bovine fetal serum which had been heated at 56 °C for 30 minutes. Then, a concentration of cells was adjusted to $5 \times 10^6$/mL with an RPMI 1640 medium containing a 10% bovine fetal serum which had been heated at 56 °C for 30 minutes, 20 mmol/L of 4-(2-hydroxyethyl)-1-piperazine ethanesulfonate, and 30 μg/mL of gentamicin. The resulting cell suspension was used as an effector cell.

The YAC-1 cell used as a target cell was maintained in an RPMI 1640 medium containing a 10% bovine fetal serum which had been heated at 56 °C for 30 minutes, at Biomedical Research Laboratories, Kureha Chemical Industry Co. Ltd. The YAC-1 cells were reacted with radioactive sodium chromate (Amersham Japan) at 37 °C for 20 minutes. Unreacted radioactive sodium chromate was removed by washing three times with an RPMI 1640 medium containing a 10% bovine fetal serum which had been heated at 56 °C for 30 minutes, and a concentration of tumor cells labeled with radioactive chromium was adjusted to $5 \times 10^4$/mL.

0.1 mL of the effector cell suspension or a double-diluted series thereof and 0.1 mL of the suspension of tumor cells labeled with radioactive chromium were put into a test tube, and reacted in a 5% carbon dioxide gas incubator at 37 °C for 4 hours. In this connection, to calculate a "specific lysis" described below, a suspension prepared by putting the tumor cells labeled with radioactive chromium and a medium into a test tube, and a suspension prepared by putting the tumor cells labeled with radioactive chromium and a detergent (Triton; a final concentration = 0.05%) into a test tube were also reacted in a 5% carbon dioxide gas incubator at 37 °C for 4 hours. After the reaction was completed, 1.5 mL of an RPMI 1640 medium containing a 10% bovine fetal serum, which had been heated at 56 °C for 30 minutes, was added to each test tube, and thoroughly mixed by a mixer. The whole was centrifuged at 12,000 rpm for 5 minutes at 4 °C to obtain a supernatant, and the radioactivity was measured by a gamma counter.

A specific lysis (S.L.) was calculated by an equation:

$$[S.L.] = \{(B - B_f)/(B_{max} - B_f)\} \times 100$$

wherein S.L. is a specific lysis (unit = %), B is a radioactivity (unit = Bq) of a supernatant of an experimental group, $B_f$ is a radioactivity (unit = Bq) of a supernatant of a spontaneously releasing group, and $B_{max}$ is a radioactivity (unit = Bq) of a supernatant of a maximum releasing group. The spontaneously releasing group means a group of culturing only the tumor cells labeled with radioactive chromium, and the maximum releasing group means a group of culturing tumor cells labeled with radioactive chromium treated with Triton. The NK cell activity was represented by "Lytic Units 30% (LU30)", that is, a number of cells which kill 30% tumor cells per $10^7$ cells of effector cells.

The results are shown in Table 6. As a control (a normal group), the above procedures were repeated, except that distilled water was orally administered for 10 days, instead of the aqueous solution of the sample for evaluation, and that the restraint stress for 18 hours was not loaded. Further, as a test for comparison, the above procedures were repeated, except that distilled water was orally administered for 10 days, instead of the aqueous solution of the sample for evaluation. In the column "Restraint" of Table 6, the symbols "(-)" and "(+)" mean "without restraint stress" and "with restraint stress", respectively.

According to a significant test, the comparative group showed P<0.01 with respect to the control group, and the M2 fraction-administered group showed P<0.05 with respect to the comparative group.

Table 6

| Group | Restraint | NK activity (LU30) |
|---|---|---|
| Control | (-) | 46.6±4.2 |
| Comparative | (+) | 28.7±2.4 |
| M2 fraction-adminstered | (+) | 34.8±4.4 |

Example 9: Examination of physicochemical properties of the adsorption fraction M2

Physicochemical properties of the M2 fraction prepared in Example 1 and the m2 fraction, prepared in Referential Example 1 as described below, from fruit bodies of commercially available *Tricholoma matsutake* were examined. Measuring methods and the results will be described below.

(1) Determination of carbohydrates

Carbohydrate content in the M2 fraction was determined by colorimetry using a phenol-sulfuric acid method. The content of carbohydrates in the M2 fraction was 62% in glucose equivalent.
The above procedure described in Example 1 was repeated twice to obtain two kinds of M2 fraction. The contents of carbohydrates therein were 69% and 70%, respectively, determined by a similar method.
The content of carbohydrates in the m2 fraction was 35% in glucose equivalent.
According to an iodostarch reaction, the M2 fraction and m2 fraction were negative. The results suggested that carbohydrates other than starch were contained.

(2) Determination of proteins

Protein content in the M2 fraction was determined by colorimetry using a copper-Folin method. The content of proteins therein was 38% in albumin equivalent.
The above procedure described in Example 1 was repeated twice to obtain two kinds of M2 fraction. The contents of proteins therein were 31% and 30%, respectively, determined by a similar method.
The content of proteins in the m2 fraction was 65% in albumin equivalent.

(3) Analysis of the carbohydrate composition

Into a tube, 1.0 mg of the M2 fraction and 0.2 mL of 2 mol/L trifluoroacetic acid were charged, and hydrolyzed at 100 °C for 6 hours. The reaction mixture was dried under a reduced pressure by an evaporator to obtain a residue. The residue was dissolved in 500 μL of purified water, and further diluted to a double volume or a ten-fold volume with purified water. To 50 μL of this solution, 500 ng of heptose was added as an internal standard substance, and the solution was applied to a high performance liquid chromatograph LC-9A (Shimadzu) equipped with a column TSKgel Sugar AXGLC-9A (15 cm x 4.6 mm ID; Tosoh) and a spectrophotometer RF-535 (Shimadzu) as a detector. The column temperature was 70 °C. The mobile phase was a 0.5 M potassium borate buffer (pH 8.7), and the flow rate thereof was 0.4 mL/min. For the conditions of post-column labeling, 1% arginine/3% boric acid was used as a reaction reagent, the flow rate was 0.5 mL/min., the reaction temperature was 150 °C, and the wavelengths for detection were EX 320 nm and EM 430 nm.
The carbohydrate composition in the M2 fraction was as follows:

Glucose 61 μg/mg, mannose 3.3 μg/mg, and galactose 2.0 μg/mg, in the order of descending content.

The carbohydrate composition in the m2 fraction was as follows:

Glucose 12.9 μg/mg, galactose 12.6 μg/mg, mannose 5.6 μg/mg, fucose 3.5 μg/mg, and xylose 0.4 μg/mg, in the order of descending content.

(4) Analysis of the amino acid composition

Acidic hydrolysis was carried out as follows. Into a tube, 0.33 mg of the M2 fraction and 0.2 mL of 6 mol/L hydrochloric acid were charged, and hydrolyzed at 110 °C for 22 hours. The reaction mixture was dried under a reduced pressure by an evaporator to obtain a residue. The residue was dissolved in 0.5 mL of purified water, and 50 μL of the solution was used for an amino acid analysis.

Alkaline hydrolysis for analyzing tryptophan was carried out as follows. After 0.48 mg of the M2 fraction was added to a plastic tube, 100 μL of 1% n-octyl alcohol-4.2 mol/L sodium hydrate solution containing 5 mg of starch soluble were further added thereto. After the plastic tube was placed into a glass tube, hydrolysis was carried out in the sealed and vacuum tube at 110 °C for 16 hours. The glass tube was cooled in air and opened. After the plastic tube was cooled in water, 1.0 mol/L.hydrochloric acid was added thereto to neutralize the mixture. Into the tube, 840 μL of purified water was added to adjust the total volume to 1000 μL, and 50 μL thereof was used for an amino acid analysis.

The quantitative determination was performed by a ninhydrin colorimetry using an amino acid analyzer L-8500 (Hitachi) as equipment.

The amino acid composition was as follows:

Aspartic acid and asparagine 10.35 mol%, threonine 5.83 mol%, serine 6.27 mol%, glutamic acid and glutamine 10.49 mol%, glycine 8.55 mol%, alanine 9.19 mol%, valine 6.88 mol%, 1/2-cystine 0.60 mol%, methionine 1.49 mol%, isoleucine 5.36 mol%, leucine 9.25 mol%, tyrosine 2.55 mol%, phenylalanine 4.05 mol%, lysine 5.17 mol%, histidine 2.18 mol%, arginine 4.44 mol%, tryptophan 1.82 mol%, and proline 5.54 mol%.

(5) Analysis of the isoelectric point

The M2 fraction was adjusted to 1 mg/mL. To (a) a mixture of 10 μL of the M2 fraction solution and 10 μL of purified water or (b) 20 μL of the M2 fraction solution (about 1.14 μg as an amount of proteins), saccharose was added so that the concentration thereof became approximately 40% (volume/volume), and then an electrophoresis was performed. The conditions of the electrophoresis were as follows:

Gel: IEF-PAGEmini (4%, pH 3-10; Tefco).
Buffer for electrophoresis: (cathode) 0.04 mol/L sodium hydroxide solution, (anode) 0.01 mol/L phosphate solution.
Conditions for electrophoresis: The electrophoresis was performed at 100 V for 30 minutes, at 300 V for 20 minutes, and at 500 V for 40 minutes.
PI marker: Each band was 1.35 g (Pharmacia).
Staining: Silver staining.

A main band was around 5.85.

(6) Nuclear magnetic resonance analysis (NMR)

The conditions for measurements were as follows.

(i) $^1$H one-dimensional NMR measurement

After 800 μL of $D_2O$ was added to 7 mg of the M2 fraction, ultrasonication was carried out for about 5 minutes in an attempt to dissolve it. The whole was centrifuged, and the supernatant was used for measurement. The conditions of the measurement were as follows.

As a detector, UNITY INOVA 600 (Varian) was used. An observation frequency was 599.6 MHz ($^1$H nucleus). As a solvent, $D_2O$ solution (concentration: saturated solution) was used. TSP 0.00 ppm ($^1$H) was used as a standard. The temperature was 25 °C. The repetition time was 7.0 seconds ($^1$H nucleus). The number of accumulation was 256.

The resulting spectrum is shown in Fig. 7. Strong signals derived from carbohydrates were observed between 3.0-5.6 ppm. It was presumed that the M2 fraction contained many carbohydrates, because the signal intensities from carbohydrates were much stronger than those from amino acids. In this connection, it is known that the signals observed between 0.5-3.0 ppm are derived from side chains of amino acids. Further, NMR signals from aromatic amino acids were observed between 6.6-7.6 ppm.

The estimated content of α glucan was 71%.

(ii) $^{13}$C one-dimensional NMR measurement

The M2 fraction was dissolved in $D_2O/CD_3OD$ (725/25), so that a concentration became approximately 20.5 mg/0.75 mL. The conditions of the measurement were as follows.

The measurement was performed at the frequency of 125.8 MHz. The standard was a deuterated methanol

($\delta$ = 49 ppm). The temperature was 45 °C. The observation width was 31.4 KHz. The data point was 64 K. The pulse width was about 41°. The repetition time of pulse was 2.5 seconds. The number of accumulation was 4000. Measurement of decoupling was performed under conditions of $^1$H complete decoupling.

The results are shown in Figs. 8 and 9. Signals from carbohydrates and signals from amino acids were observed. The signal intensities from carbohydrates were stronger than those from amino acids. Because the major component of carbohydrates contained in the M2 fraction is glucose, it was considered that signals around 95-110 ppm were derived from a carbon at the 1-position of glucose, signals around 105 ppm were from a carbon at the $\beta$1 position, and signals around 102 ppm and around 99 ppm were from a carbon at the $\alpha$1 position. From the results, at least three or more binding types were presumed. Signals around 63 ppm were derived from the 6-position. The fact that three signals were observed around 63 ppm supports the above presumption, i.e., the M2 fraction has three or more binding types. Further, it is considered from signals around 70-80 ppm that the 4-position is involved in binding, and it is presumed that an $\alpha$1-4 bond and a $\beta$1-4 bond are contained in the M2 fraction.

(7) Circular dichroism analysis (CD)

Water was added to approximately 3 mg of the M2 fraction, so that a concentration became 2 mg/mL. Because a precipitate was observed, the whole was centrifuged, and the supernatant was used for measurement. The conditions for measurement were as follows.

As a detector, JASCOJ-500A was used. As a solvent, water was used. The concentration of proteins was approximately 2 mg/mL. The wavelength area was 200 to 250 nm. The cell length was 1 mm. The temperature was room temperature (approximately 23 °C). The number of accumulation was 8. The measurement was performed under the above conditions.

The resulting CD spectrum is shown in Fig. 10. The CD value (vertical axis) is ellipse angle (mdeg). It was presumed that secondary structures such as $\alpha$-helix were a minor structure, and that unordered structure was a major structure.

(8) Optical rotation

Optical rotation measured at 25 °C was 42.

(9) Infrared spectroscopic analysis

Infrared spectroscopic analysis was carried out by a KBr method. More particularly, 0.5 mg of the M2 fraction was uniformly mixed with 15 mg of KBr powder, and disks were formed by pressing and measured.

The resulting spectrum is shown in Fig. 11. This spectrum suggested that polysaccharides were contained in the M2 fraction.

(10) Ultraviolet spectroscopic analysis (UV)

The M2 fraction was dissolved in purified water (concentration = 0.5 mg/10 mL) and a UV was measured. As a detector, 2500PC (Shimadzu) was used.

The resulting ultraviolet and visible absorption spectrum is shown in Fig. 12. A weak absorption maximum was detected at 260 to 270 nm.

(11) Electron spin resonance (ESR)

The ESR of the sample was measured under a nitrogen atmosphere using ESP350E (Brucker). The conditions of the measurement are shown in Table 7.

The results are shown in Table 8 and Figs. 13 and 14. In Figs. 13 and 14, the phrase "Intensity (arb. units)" in the vertical axis means that the unit of "intensity" shown at the vertical axis is arbitrary. A signal, which was considered to be derived from carbon radicals, was observed around g = 2.004. Further, it was considered that signals around g = 4.25 ($Fe^{3+}$) and around g = 2.03-2.05 were derived from transition metal ions.

Table 7

| Conditions | Broad | Around g = 2 |
|---|---|---|
| Measuring temperature | Room temperature | Room temperature |
| Magnetic field sweep area | $0\sim1$ T | 339.0-359.0 mT |
| Modulation | 100 kZ, 0.5 mT | 100 kZ, 0.2 mT |
| Microwave | 10 mW, 9.79 GHz | 0.2 mW, 9.79 GHz |
| Sweep time | 167.772 s $\times$ 1 time | 83.886 s $\times$ 10 times |
| Time constant | 163.84 ms | 163.84 ms |
| Number of data points | 4096 points | 2048 points |
| Cavity | $TM_{110}$, cylinder | $TM_{110}$, cylinder |

Table 8

| Index | M2 fraction | m2 fraction |
|---|---|---|
| g value | 2.0042 | 2.0039 |
| Line width (mT) | 0.68 | 0.67 |
| Spin density (spins/g) | $4.7\times10^{16}$ | $2.8\times10^{16}$ |

(12) Viscosity

After 0.5 g of the sample (the M2 fraction or m2 fraction) was dissolved in 100 mL of purified water, and the whole was centrifuged at 10000 rpm. The supernatant was adjusted to 1.67 mg/mL by adding purified water, and a reduced viscosity was measured at 30 °C by an Ostwald viscometer. The reduced viscosities of the M2 and m2 fractions were 108 $\eta$ and 924 $\eta$, respectively.

(13) Molecular weight

After the sample (the M2 fraction or m2 fraction) was dissolved in purified water, so that the concentration was 2-3 mg/mL, gel filtration was carried out.
In the gel filtration, a feed pump LC-7A (Shimadzu) was used as equipment, an ultraviolet spectrophotometer detector SPD-6A (Shimadzu) was used as a detector, and TSKgel G3000SW (7.5 mm I.D. x 30 cm; Tosoh) was used as a column. Further, the column temperature was room temperature, the mobile phase was 50 mmol/L phosphate buffer (pH 7.0) containing 0.15 mol/L sodium sulfate, the flow rate thereof was 0.8 mL/min, and the wavelength for detection was 214 nm. A molecular weight was calculated by extrapolating an elution time to a standard curve prepared from compounds with a known molecular weight.
In the M2 fraction, the molecular weight of the major component was.2000 kDa, and other components of 4.0 kDa and 1.2 kDa were confirmed. In the m2 fraction, the molecular weight of the major component was 2000 kDa, and other components of 7.0 kDa and 1.0 kDa were confirmed.

(14) Elemental analysis

Carbon (C), hydrogen (H), and nitrogen (N) were measured using CHN coder TM-5 (Yanako).
As to sulfur (S), phosphorus (P), and chlorine (Cl), after the sample was combusted and decomposed, $SO_4^{2-}$, $PO_4^{3-}$, and $Cl^-$ in an absorbing solution were measured by an ion chromatography (IC) method, and the resulting values were converted to those of the elements. More particularly, 1 mL of acetone was added to 0.1 g of the sample. After an introduction of oxygen (3 Mpa), the whole was subjected to combustion and cooled in water for 30 minutes. After an absorbing solution (0.1mol/L NaOH) and a washing solution were combined and adjusted to 100 mL, the measurement was carried out using Dionex DX-300 type IC.
The results are shown in Table 9.

Table 9

|  | Content (%) | |
| --- | --- | --- |
| Elements | M2 fraction | m2 fraction |
| carbon | 41.3 | 40.4 |
| hydrogen | 6.0 | 6.0 |
| nitrogen | 5.1 | 8.0 |
| sulfur | 1.0 | 0.22 |
| phosphorus | 0.052 | 0.096 |
| chlorine | 0.16 | 0.13 |

(15) Estimated content of $\alpha$-glucan

After the sample (the M2 fraction or m2 fraction) was dissolved in 0.5 mol/L of an acetate buffer (pH 4.3), an amyloglucosidase solution (Sigma Chem. Co., USA) was added to the solution. After the whole was shaken at 60 °C for 30 minutes and adjusted to pH 4.5, glucoamylase was added. The whole was further shaken at 60 °C for 30 minutes. After the reaction, an amount of glucose in the resulting solution was measured by a glucose analyzer. The value obtained by subtracting the measured value from an amount of glucose in a blank solution was regarded as an "estimated amount of $\alpha$-glucan".

In addition, after 1.0 mol/L of sulfuric acid was added to the sample, the whole was hydrolyzed at 100 °C for 18 hours, and then neutralized. An amount of glucose in the resulting solution was measured by a glucose analyzer, and the value was regarded as a "total amount of glucan". The estimated content of $\alpha$-glucan was calculated as a percentage of the "estimated amount of $\alpha$-glucan" with respect to the "total amount of glucan".

The estimated content of $\alpha$-glucan in the M2 fraction was 71% with respect to all carbohydrates, and that in the m2 fraction was 32% with respect to all carbohydrates.

(16) Determination of endotoxin

An amount of endotoxin was determined by a LAL (Limulus Amoebocyte Lysate) reaction (Ohbayashi T. et al., Clin.Chim.Acta, 149, 55-65, 1985) using a commercially available measuring kit (Endospecy; Seikagaku Corp.), equipment (endotoxin-free), and reagents (Seikagaku Corp.).

More particularly, after the M2 fraction was dissolved in distilled water, so that the concentration became an appropriate concentration, 50 µL of the aliquot was added to a well in a 96-well microplate (endotoxin-free). The same volumes of a diluted series of an endotoxin standard solution or distilled water were added to other wells of the microplate. Then, 50 µL of an LAL solution (a reagent from Limulus) was added to each well thereof. After incubation at 37 °C for 30 minutes, a diazo coupling reagent was added. After color development, absorbance at 545 nm (control = 630 nm) was measured. An amount of endotoxin in the M2 fraction calculated from a calibration curve of the standard solution was 2.5 ng/mg.

Example 10: Presumption of active structure of the M2 fraction

In the present example, to presume an active structure of the M2 fraction, the M2 fraction was chemically or enzymatically treated, the resulting preparation was orally administered to mice to which tumor cells had been implanted at a cecal wall, and the activity of cells from a mesenterium lymph node was evaluated by MLR and MLR-CMC.

More particularly, a peptide portion of the M2 fraction was prepared according to a method of Takasaki et al. (Takasaki S. et al., Methods in Enzymology, vol.83, Academic Press, New York, 263-268, 1982) to prepare a fraction with a reduced content of proteins as follows. After the M2 fraction (carbohydrates:proteins = 68:32) was dissolved in hydrazine anhydride, the solution was added to a glass tube, and heated at 100 °C for 24 hours in the sealed tube. The resulting solution was placed in a desiccator, and allowed to stand under reduced pressure to remove hydrazine. After the dried product was dissolved in purified water, the solution was applied to gel filtration using Sephadex G-25 (Pharmacia) to collect a high molecular fraction. The resulting fraction was applied to an ionexchange chromatography using diethylaminoethyl sephacel to obtain a fraction which passed through the column (carbohydrates:proteins = 99:1).

To prepare a fraction with a reduced content of $\alpha$-glucan, the M2 fraction was dissolved in 0.5 mol/L of an acetated buffer (pH 4.3), amyloglucosidase (Sigma Chem. Co.) was added thereto, and the whole was reacted at 60 °C for 30 minutes. After the whole was adjusted to pH 4.5, glucoamylase (Wako Pure Chemical Industries) was added thereto, and the whole was reacted at 60 °C for 30 minutes. The whole was applied to gel filtration using Sephadex G-25 to collect a high molecular fraction (carbohydrates:proteins = 13:87). The decrease of $\alpha$-glucan was confirmed by an NMR analysis.

Further, to prepare a fraction with reduced $\beta$-glucan, the M2 fraction was dissolved in 0.5 mol/L of an acetated buffer (pH 4.3), $\beta$-glucosidase (Sigma Chem. Co.) was added thereto, and the whole was reacted at 37 °C for 24 hours. The whole was applied to gel filtration using Sephadex G-25 to collect a high molecular fraction (carbohydrates:proteins = 59:41). The decrease of $\beta$-glucan was confirmed by an NMR analysis.

The activity of cells from a mesenterium lymph node was evaluated in a manner similar to that in Example 5.

The results are shown in Table 10. The "control group" in Table 10 means a control group in which distilled water was administered. A significant test was carried out with respect to the control group, and "NS" means "Not Significant".

As apparent from Table 10, when the peptide portion in the M2 fraction was treated with hydrazine to decompose and remove the peptide portion, the activity clearly disappeared. Further, when the carbohydrate portion in the M2 fraction was treated with amyloglucosidase and glucoamylase to reduce $\alpha$-glucan, the activity was decreased, but the activity was not affected by the $\beta$-glucosidase treatment.

The results suggested that the active structure of the M2 fraction was $\alpha$-glucan and proteins.

Table 10

| Treatment to M2 fraction | MLR-CMC (% to control group) | Significant test |
|---|---|---|
| Non-treated | 177±32 | p<0.01 |
| Hydrazine treatment | 112±19 | NS |
| Amyloglucosidase and glucoamylase treatment | 137±27 | NS |
| β-glucosidase treatment | 165±25 | p<0.01 |

Referential Example 1: Preparation of an adsorption fraction of an extract mixture from commercially available *Tricholoma matsutake* by an anion exchange resin

After 100 g of commercially available *Tricholoma matsutake* fruit bodies harvested in Nagano Prefecture was lyophilized to remove water, the fruit bodies were crushed to obtain 15 g of powder.

The extraction and fractionation procedures in Example 1 was repeated, except that the fruit body powder was used instead of the mycelia as a starting material, to obtain a non-adsorption fraction ml and an adsorption fraction m2.

INDUSTRIAL APPLICABILITY

The immune enhancing agent of the present invention can enhance an immune activity in a subject in need of an immune enhancement, and is effective, for example, in treating or preventing cancer, particularly a primary tumor or metastatic foci. Further, the agent of the present invention for promoting a recovery from stress can promote such a recovery from stress.

Although the present invention has been described with reference to specific embodiments, various changes and modifications obvious to those skilled in the art are possible without departing from the scope of the appended claims.

**Claims**

1. An adsorption fraction of a liquid mixture by an anion exchange resin, wherein the liquid mixture is obtainable by mixing a hot water extract of a mycelium of *Tricholoma matsutake* FERM BP-7304 with an alkaline solution extract of a mycelial residue obtained when preparing the hot water extract, and

   (a) the content of carbohydrates in the adsorption fraction is 60 to 72% as a glucose equivalent determined by a phenol-sulfuric acid method, and
   (b) the content of proteins in the adsorption fraction is 28 to 40% as an albumin equivalent determined by a copper-Folin method.

2. An immune enhancing agent comprising as an active ingredient the adsorption fraction according to claim 1.

3. An immune enhancing composition comprising the adsorption fraction according to claim 1 and a pharmaceutically acceptable carrier.

4. An immune enhancing health food comprising the adsorption fraction according claim 1, alone or, optionally, with one or more food components.

5. The immune enhancing health food according to claim 4, wherein the health food is a functional food.

6. A method for an immune enhancement, comprising administering to a subject in need thereof the adsorption fraction according to claim 1 in an amount effective therefor.

7. Use of the adsorption fraction according to claim 1 in the manufacture of an immune enhancing composition or health food.

8. An agent for treating or preventing metastatic foci, comprising as an active ingredient the adsorption fraction according to claim 1.

9. A composition for treating or preventing metastatic foci, comprising the adsorption fraction according to claim 1 and a pharmaceutically acceptable carrier.

10. A health food for treating or preventing metastatic foci, comprising the adsorption fraction according to claim 1, alone or, optionally, with one or more food components.

11. The health food according to claim 10, which is a functional food.

12. A method for treating or preventing metastatic foci, comprising administering to a subject in need thereof the adsorption fraction according to claim 1 in an amount effective therefor.

13. Use of the adsorption fraction according to claim 1 in the manufacture of a composition or health food for treating or preventing metastatic foci.

14. An agent for increasing a serum IAP value, comprising as an active ingredient the adsorption fraction according to claim 1.

15. A composition for increasing a serum IAP value, comprising the adsorption fraction according to claim 1 and a pharmaceutically acceptable carrier.

16. A health food for increasing a serum IAP value, comprising the adsorption fraction according to claim 1, alone or, optionally, with one or more food components.

17. The health food according to claim 16, which is a functional food.

18. A method for increasing a serum IAP value, comprising administering to a subject in need thereof the adsorption fraction according to claim 1 in an amount effective therefor.

19. Use of the adsorption fraction according to claim 1 in the manufacture of a composition or health food for increasing a serum IAP value.

20. An agent for promoting a recovery from stress, comprising as an active ingredient the adsorption fraction according to claim 1.

21. A composition for promoting a recovery from stress, comprising the adsorption fraction according to claim 1 and a pharmaceutically acceptable carrier.

22. A health food for promoting a recovery from stress, comprising the adsorption fraction according to claim 1, alone or, optionally, with one or more food components.

23. The health food according to claim 22, which is a functional food.

24. A method for promoting a recovery from stress, comprising administering to a subject in need thereof the adsorption fraction according to claim 1 in an amount effective therefor.

25. Use of the adsorption fraction according to claim 1 in the manufacture of a composition or health food for promoting a recovery from stress.

## F I G. 1

## F I G. 2

F I G. 3

F I G. 4

F I G. 5

F I G. 6

FIG. 7

F I G.  8

110    105    100    95    90    85    80    75    70    65    ppm

F I G.  9

220   200   180   160   140   120   100   80   60   40   20   ppm

F I G. 1 0

F I G. 1 1

Transmittance (%) vs Wave number (cm$^{-1}$)

F I G． 1 2

F I G． 1 3

g=2.0042

F I G. 1 4

## INTERNATIONAL SEARCH REPORT

| | International application No. |
|---|---|
| | PCT/JP03/01979 |

A. CLASSIFICATION OF SUBJECT MATTER
   Int.Cl$^7$  A61K35/84, A61P35/00, 35/04, 37/04, 43/00, A23L1/30

According to International Patent Classification (IPC) or to both national classification and IPC

B. FIELDS SEARCHED

Minimum documentation searched (classification system followed by classification symbols)
   Int.Cl$^7$  A61K35/84, A61P35/00, 35/04, 37/04, 43/00, A23L1/30

Documentation searched other than minimum documentation to the extent that such documents are included in the fields searched

Electronic data base consulted during the international search (name of data base and, where practicable, search terms used)
   CAPLUS(STN), BIOSIS(STN), EMBASE(STN), MEDLINE(STN), JICST(JOIS)

C. DOCUMENTS CONSIDERED TO BE RELEVANT

| Category* | Citation of document, with indication, where appropriate, of the relevant passages | Relevant to claim No. |
|---|---|---|
| Y | WO 01/49308 A1 (KUREHA CHEMICAL INDUSTRY CO., LTD.), 12 July, 2001 (12.07.01), & EP 1256351 A1  & US 2003/044424 A1 & AU 2404601 A | 1-5,7-11, 13-17,19-23, 25 |
| P,Y | WO 02/30440 A1 (KUREHA CHEMICAL INDUSTRY CO., LTD.), 18 April, 2002 (18.04.02), & AU 2001094210 A | 1-5,7-11, 13-17,19-23, 25 |
| E,Y | Takusaburo EBINA et al., Antitumor Effect of a Peptide-Glukan Preparation Extracted from Mycelium of Tricholoma Matsutake, Biotherapy, 2002.May, pages 255 to 259, Summary | 1-5,7-11, 13-17,19-23, 25 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 02 May, 2003 (02.05.03) | 20 May, 2003 (20.05.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.

PCT/JP03/01979

C (Continuation). DOCUMENTS CONSIDERED TO BE RELEVANT

| Category* | Citation of document, with indication, where appropriate, of the relevant passages | Relevant to claim No. |
|---|---|---|
| E,Y | Takusaburo EBINA, "Tanshikin Matsutake Kinshitai Chushutsubutsu no Koshuyo Koka", Japanese journal of bacteriology, 28 February, 2002 (28.02.02), Vol.57, No.1, page 259 | 1-5,7-11, 13-17,19-23, 25 |
| Y | Ha W.K. et al., Screening of edible Japanese Plants for suppressive effects on phorbol ester-induced superoxide generation in differentiated HL-60 cells and AS52 cells, Cancer Letters, 08 February, 2002 (08.02.02), pages 7 to 16 | 1-5,7-11, 13-17,19-23, 25 |

Form PCT/ISA/210 (continuation of second sheet) (July 1998)

**INTERNATIONAL SEARCH REPORT**

International application No.

PCT/JP03/01979

**Box I    Observations where certain claims were found unsearchable (Continuation of item 2 of first sheet)**

This international search report has not been established in respect of certain claims under Article 17(2)(a) for the following reasons:

1. ☒ Claims Nos.: 6, 12, 18, 24

because they relate to subject matter not required to be searched by this Authority, namely:

The inventions as set forth in claims 6, 12, 18, 24 pertain to method for treatment of the human body by therapy and thus relate to a subject matter which this International Searching Authority is not required, under the provisions of Article 17(2)(a)(i) of the PCT (continued to extra sheet)

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.

☐   No protest accompanied the payment of additional search fees.

Form PCT/ISA/210 (continuation of first sheet (1)) (July 1998)

| INTERNATIONAL SEARCH REPORT | International application No. |
|---|---|
| | PCT/JP03/01979 |

Continuation of Box No.I-1 of continuation of first sheet(1)

and Rule 39.1(iv) of the Regulations under the PCT, to search.

Form PCT/ISA/210 (extra sheet) (July 1998)